(19) **Europäisches Patentamt**
**European Patent Office**
**Office européen des brevets**

(11) **EP 3 844 021 B1**

(12) **EUROPÄISCHE PATENTSCHRIFT**

(45) Veröffentlichungstag und Bekanntmachung des Hinweises auf die Patenterteilung:
**15.01.2025  Patentblatt 2025/03**

(21) Anmeldenummer: **19759363.5**

(22) Anmeldetag: **27.08.2019**

(51) Internationale Patentklassifikation (IPC):
**B60N 2/56** (2006.01)

(52) Gemeinsame Patentklassifikation (CPC):
**B60N 2/5642; B60N 2/56; B60N 2/5678; B60N 2/90**

(86) Internationale Anmeldenummer:
**PCT/EP2019/072748**

(87) Internationale Veröffentlichungsnummer:
**WO 2020/043679 (05.03.2020 Gazette 2020/10)**

(54) **FAHRZEUGSITZ UND EIN VERFAHREN ZUM BETRIEB EINES FAHRZEUGSITZES**

VEHICLE SEAT AND METHOD FOR OPERATING A VEHICLE SEAT

SIÈGE DE VÉHICULE ET PROCÉDÉ SERVANT À FAIRE FONCTIONNER UN SIÈGE DE VÉHICULE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.08.2018  DE 102018120996**
**20.08.2019  DE 102019122278**

(43) Veröffentlichungstag der Anmeldung:
**07.07.2021  Patentblatt 2021/27**

(73) Patentinhaber: **GRAMMER AG**
**92289 Ursensollen (DE)**

(72) Erfinder:
• **ÜBELACKER, Roland**
**92536 Pfreimd (DE)**
• **KRIVENKOV, Konstantin**
**92224 Amberg (DE)**

(74) Vertreter: **Hannke Bittner & Partner mbB**
**Regensburg**
**Prüfeninger Straße 1**
**93049 Regensburg (DE)**

(56) Entgegenhaltungen:
DE-A1- 102013 003 673    DE-A1- 102016 219 203
US-A- 5 934 748          US-A1- 2006 244 289
US-B2- 6 892 807         US-B2- 9 511 646

**Beschreibung**

Die Erfindung betrifft einen Sitz für ein Fahrzeug und ein Verfahren zum Betrieb eines Sitzes für ein Fahrzeug.

Bei herkömmlichen Fahrzeugsitzen ergibt sich oft das Problem einer unzureichenden Luftzirkulation zwischen dem Sitz und der sich auf dem Sitz befindlichen Person. Feuchtigkeit insbesondere Schweiß kann in den Kontaktbereichen des Nutzers mit dem Sitz somit nicht mehr ausreichend durch die Umgebungsluft absorbiert werden. Dies kann insbesondere bei längeren Fahrten auftreten, beispielsweise bei Sitzen in Nutzfahrzeugen, in denen sich der Nutzer bekanntlich lange aufhält.

Die Körperflüssigkeit wird dann durch den Sitz beziehungsweise das Polster aufgenommen. Ein feuchter Sitz kann beim Nutzer als unangenehm empfunden werden. Darüber hinaus ist das Feuchtigkeitsmilieu fördernd für bakterielles Wachstum.

Herkömmliche Sitze weisen oft eine Sitzheizung oder eine Belüftungseinrichtung auf, welche durch den Nutzer aktiviert werden. Der Nutzer ist jedoch bei der Aktivierung meistens bereits unterkühlt, verschwitzt, feucht oder nass, wodurch die Feuchtigkeit durch den Sitz beziehungsweise die Sitzpolster aufgenommen wird. Ferner kommt es oft vor, dass belüftete Sitze die Hautoberfläche zu sehr unterkühlen, wodurch Verspannungen oder Rückenbeschwerden bei dem Nutzer verursacht werden können.

Ziel der Erfindung ist es demnach einen Sitz für ein Fahrzeug beziehungsweise ein Verfahren zum Betrieb eines Sitzes für ein Fahrzeug bereitzustellen, welche die eingangs benannten Probleme lösen.

Der Sitz umfasst erfindungsgemäß zumindest eine Sensoreinrichtung. Die Sensoreinrichtung weist zumindest einen Temperatursensor und zumindest einen Feuchtigkeitssensor auf, welche die Komfortparameter Temperatur und/oder Feuchtigkeit in dem Sitz insbesondere an oder nahe der Oberfläche des Sitzes erfassen können. Ferner ist es von Vorteil, wenn die Komfortparameter unter der Oberfläche oder inmitten des Polsteraufbaus erfasst werden können. Bei Sitzbelegung ist diese Oberfläche vorzugsweise in Kontakt mit dem Nutzer. Die Sensoreinrichtung kann jedoch auch noch weitere Sensoren umfassen, mittel welchen Sensordaten verschiedenster Art detektiert werden können.

Der Sitz weist erfindungsgemäß weiterhin eine Regel-/ Steuereinrichtung auf, welche das Sitzklima anhand der Komfortparameter steuert, beziehungsweise regelt. Die Sensoreinrichtung ist demnach signaltechnisch mit der Regel-/ Steuereinrichtung verbunden. Erfindungsgemäß ist das Sitzklima aktiv mittels der Regel-/ Steuereinrichtung steuerbar beziehungsweise regelbar, wobei unter der Regelung/Steuerung des Sitzklimas die Regelung/Steuerung der Komfortparameter zu verstehen ist.

Unter einer Steuerung wird üblicherweise verstanden, dass aufgrund eines Eingangssignals eine Ausgangsgröße folgt. Ein solches Eingangssignal ist zumeist eine binäre Größe, beispielsweise Ein/Aus. Eine Regelung basiert auf einer Rückkopplung eines Ausgangssignals. Bei einer Regelung wird die Regelgröße kontinuierlich mit einem Sollwert verglichen. Der Regler bestimmt entsprechend der Abweichung der Werte eine Stellgröße, die so auf die Regelgröße einwirkt, dass sie die Abweichung minimiert und die Regelgröße ein gewünschtes Zeitverhalten annimmt, trotz vorhandener Störgrößen.

Erfindungsgemäß steuert beziehungsweise regelt die Regel-/ Steuereinrichtung eine Heizeinrichtung und eine Lüftungseinrichtung des Sitzes, wodurch das Sitzklima entsprechend aktiv regelbar ist.

Die Aufgabe der Erfindung wird demnach durch den Gegenstand des Anspruchs 1 gelöst.

Vorteilhafte Ausführungsformen sind in den Unteransprüchen offenbart.

Ein wesentlicher Punkt der Erfindung ist ein Fahrzeugsitz umfassend zumindest ein Sitzelement, in welchem zumindest eine Heizeinrichtung und zumindest eine Belüftungseinrichtung vorgesehen ist, wobei der Fahrzeugsitz eine Sensoreinrichtung umfasst, welche zumindest einen Temperatursensor und zumindest einen Feuchtigkeitssensor aufweist, wobei eine Regel-/ Steuereinrichtung anhand der Daten der Sensoreinrichtung bezüglich der Komfortparameter Temperatur und/oder Feuchtigkeitsgehalt die Heizeinrichtung und/oder die Belüftungseinrichtung steuert und/oder regelt, wodurch das Sitzklima aktiv steuerbar und/oder regelbar ist.

Durch einen derartigen Fahrzeugsitz wird das Sitzklima bereits entsprechend den Komfortparametern angepasst, bevor der Nutzer die unangenehmen Folgen eines nicht optimalen Sitzklimas verspürt. Derartige unangenehme Folgen sind wie bereits angeführt Unterkühlung, Verschwitztheit, Feuchtigkeit oder Nässe, wodurch Verspannungen oder Rückenbeschwerden bei dem Nutzer verursacht werden können.

Natürlich können unter den Begriff Komfortparameter noch weitere Daten fallen. Hierzu können auch noch weitere Sensoren vorhanden sein, welche signaltechnisch mit der Regel-/Steuereinrichtung verbunden sind. Derartige Sensoren können auch außerhalb des Fahrzeugsitzes angeordnet sein.

Denkbar wären zum Beispiel Sensoren, welche das Fahrverhalten analysieren. So könnte bei einem auffälligem Fahrverhalten, beispielsweise durch Müdigkeit, die Regel-/Steuereinrichtung bestimmte Einrichtungen aktivieren, beispielsweise den Sitz abkühlen.

Ferner kann vorteilhafterweise die Regel-/Steuereinrichtung neben der Heizeinrichtung und der Belüftungseinrichtung mit weiteren Einrichtungen signaltechnisch verbunden sein und diese entsprechend regeln oder steuern.

Denkbar wäre auch die Heizeinrichtung und/oder die Belüftungseinrichtung anhand vorgegebener Zeitpunkte anzusteuern. Dies kann beispielsweise eine morgendliche Verheizung des Sitzes an einem kalten Wintertag umfassen.

Nach einer bevorzugten Ausführungsform weist die Regel-/Steuereinrichtung eine Speichereinrichtung auf, in welcher Sollwerte oder Sollbereich für die Komfortparameter oder Sollwerte oder Sollbereiche für Komfortparameterkombinationen abgelegt sind. Diese Sollwerte stellen vorgegebene Komfortgrenzen der Komfortparameter oder der Komfortparameterkombinationen dar. Die Regel-/Steuereinrichtung vergleicht vorteilhafterweise, die durch die Sensoreinrichtung bereitgestellten Daten mit den entsprechenden Sollwerten beziehungsweise Komfortgrenzen. Eine aktive Regelung der Komfortparameter beziehungsweise des Sitzklimas erfolgt dann vorzugsweise durch ein automatisches Ansteuern der Heizeinrichtung und/oder der Belüftungseinrichtung durch die Regel- Steuereinrichtung. Vorteilhafterweise steuert/regelt die Regel-/ Steuereinrichtung individuelle Komfortparameter. Demnach kann beispielsweise ein bestimmter Temperaturwert oder Feuchtigkeitsgehalt eingestellt werden. Oftmals ist es jedoch für eine optimale Sitzklimatisierung notwendig, bestimmte Komfortparameterkombinationen vorzugeben. Demnach wäre bei einem bestimmten Feuchtigkeitsgehalt ein bestimmter vorgebbarer Temperaturbereich oder eine bestimmte vorgegebene Temperatur optimal. Derartige Komfortparameterkombinationen können als Sollwerte oder Sollbereiche in der Speichereinrichtung abgelegt werden.

Nach einer weiteren bevorzugten Ausführungsform ist eine Eingabeeinrichtung mit der Regel-/Steuereinrichtung signaltechnisch verbunden. Vorteilhafterweise kann der Nutzer mittels der Eingabeeinrichtung eine direkte Einstellung der Komfortparameter, beispielsweise der Temperatur oder der Feuchtigkeitsgehalt vornehmen. Es ist weiterhin von Vorteil, dass mittels der Eingabeeinrichtung die Sollwerte, beziehungsweise die Sollbereiche der Komfortparameter, beziehungsweise der Komfortparameterkombinationen mittels der Eingabeeinrichtung manipulierbar und/oder in der Speichereinrichtung eintragbar sind.

Nach einer bevorzugten Ausführungsform umfasst der Sitz zwei Sitzelemente. Vorteilhafterweise ist ein Sitzelement ein unteres Sitzteil d. h. derjenige Teil des Sitzes, auf welchem der Nutzer sitzt. Vorteilhafterweise ist das weitere Sitzelement ein Rückenlehnenelement. Dabei ist es von Vorteil, dass die Sensoreinrichtung zumindest einen Temperatursensor und zumindest einen Feuchtigkeitssensor für das untere Sitzteil und zumindest einen Temperatursensor und zumindest einen Feuchtigkeitssensor für das Rückenlehnenelement umfasst. Demnach kann das Sitzklima sowohl im unteren Sitzteil als auch in dem Rückenlehnenelement aktiv geregelt werden. Eine entsprechende Regelung kann zugleich in beiden Sitzelementen erfolgen. Alternativ kann eine aktive Regelung oder Steuerung in den individuellen Sitzelementen unabhängig voneinander erfolgen. Vorteilhafterweise ist durch die Eingabeeinrichtung auswählbar, bei welchem Sitzelement, beziehungsweise bei beiden Sitzelementen, eine aktive Sitzklimatisierung erfolgen soll.

Die Sitzelemente unteres Sitzteil und Rückenlehnenelement umfassen vorteilhafterweise jeweils eine Höhenrichtung Z, Z'. Die Höhenrichtung Z, Z' ist derart zu verstehen, dass die oberste Schicht des Sitzelements diejenige ist, welche mit dem Nutzer in Kontakt ist und das unterste Element dasjenige ist, welches am weitesten von dem Nutzer entfernt ist.

Erfindungsgemäß umfasst das zumindest eine Sitzelement eine erste Schicht, welche aus einem Material besteht, das Feuchtigkeit aufnehmen und durchleiten kann. Erfindungsgemäß ist das Material der ersten Schicht porös und/oder offenporig ausgebildet. Bevorzugt besteht die erste Schicht aus einem feuchtigkeitsleitenden Schaumstoff oder einem feuchtigkeitsleitenden Gewebe. Dieses Material ist vorteilhafterweise offenporig und ist geeignet die Feuchtigkeit sofort aufzunehmen und dann entsprechend weiterzuleiten. Es wäre auch denkbar, dass das Material der ersten Schicht ein Flies, ein Gewebe, Wirkware oder ein Abstandsgewirke ist.

Vorteilhafterweise ist die Oberfläche der ersten Schicht bei Sitzbelegung in direkten Kontakt mit dem Nutzer oder in unmittelbarer Nähe zu dem Nutzer. Es wäre weiterhin denkbar, weitere Schichten über der ersten Schicht anzuordnen, wobei diese Schichten ebenfalls feuchtigkeitsleitend sein sollten. Es kann beispielsweise vorteilhaft sein, dass diese erste Schicht von einem ebenfalls feuchtigkeitsleitenden Bezug beispielsweise einem feuchtigkeitsleitenden Stoffbezug bedeckt wird. Demnach kann der Kontakt mit dem Nutzer direkt sein oder indirekt durch den dazwischenliegenden Bezug.

Erfindungsgemäß sind zumindest ein Temperatursensor und zumindest ein Feuchtigkeitssensor in dem zumindest einem Sitzelement derart angeordnet, dass die Komfortparameter Temperatur und Feuchtigkeit an oder nahe der Oberfläche des Sitzelements erfassbar sind. Ferner ist es von Vorteil, dass die Komfortparameter Temperatur und/oder Feuchtigkeit unter der Oberfläche oder im Polsteraufbau erfassbar sind. Es kann auch von Vorteil sein, die Komfortparameter in einer bestimmten Schicht, beispielsweise der ersten Schicht zu erfassen. Erfindungsgemäß sind eine Heizeinrichtung und eine Belüftungseinrichtung in dem zumindest einem Sitzelement angeordnet, wodurch die Komfortparameter beziehungsweise das Sitzklima aktiv regelbar sind.

Nach einer weiteren bevorzugten Ausführungsform sind zumindest ein Temperatursensor und/oder zumindest ein Feuchtigkeitssensor in beiden Sitzelementen also sowohl in dem unteren Sitzteil als auch in dem Rückenlehnenelement angeordnet, wodurch die Komfortparameter Temperatur und/oder Feuchtigkeit an oder nahe den Oberflächen der Sitzelemente erfassbar sind. Ferner sind vorteilhafterweise zumindest eine Heizeinrichtung und zumindest eine Belüftungseinrichtung in den beiden Sitzelementen angeordnet, wodurch die Komfortparameter beziehungsweise das Sitzklima steuerbar/regelbar sind.

Nach einer weiteren bevorzugten Ausführungsform ist der zumindest eine Temperatursensor und/oder der zumindest eine Feuchtigkeitssensor in und/oder über und/oder unter der ersten Schicht angeordnet. Der Begriff unter der ersten Schicht kann zum einen bedeuten, dass die Sensoren sich direkt unter der ersten Schicht befinden. Es kann jedoch auch möglich sein, dass noch weitere Schichten sich zwischen der ersten Schicht und den Sensoren befinden. Analog kann der Begriff "über der ersten Schicht" ausgelegt werden. Es wäre auch denkbar, dass der zumindest eine Temperatursensor und/oder der zumindest eine Feuchtigkeitssensor in dem Bezug angeordnet ist/sind, welcher auf der ersten Schicht angeordnet ist.

Dabei ist es von Vorteil, dass die Regel-/Steuereinrichtung die von der Sensoreinrichtung übertragenen Daten in Abhängigkeit zum Abstand des jeweiligen Sensors zu der mit dem Nutzer in Kontakt stehenden Oberfläche des zumindest einen Sitzelements ausgewertet werden. Durch die vorteilhafte Berücksichtigung des Abstands zu dem Nutzer kann eine wesentlich genauere Einstellung des Sitzklimas erfolgen. Ferner wäre es denkbar, dass Materialkennwerte der zwischen dem Nutzer und dem Sensor liegenden Materialien berücksichtigt werden. Derartige Materialkennwerte können beispielsweise Aufnahme, Kapazitäten der Schichten bezüglich der Feuchtigkeit umfassen.

Nach einer bevorzugten Ausführungsform sind die Heizeinrichtung und die Belüftungseinrichtung in dem zumindest einem Sitzelement angeordnet. Vorteilhafterweise ist in Höhenrichtung Z, Z' des Sitzelements unter der ersten Schicht zumindest eine Heizeinrichtung angeordnet. Der Begriff unter der ersten Schicht kann zum einen bedeuten, dass die Heizeinrichtung sich direkt unter der ersten Schicht befindet. Es kann jedoch auch möglich sein, dass noch weitere Schichten zwischen der ersten Schicht und der Heizeinrichtung sich befinden. Es wäre denkbar, dass in Höhenrichtung des Sitzelements unter der ersten Schicht genau eine Heizeinrichtung angeordnet ist. Es wäre auch denkbar, dass zwei oder mehrere Heizeinrichtung unter der ersten Schicht angeordnet sind.

Analog ist in Höhenrichtung Z, Z' des Sitzelements unter der ersten Schicht die zumindest eine Belüftungseinrichtung angeordnet.

Es wäre auch denkbar, dass zumindest eine Heizeinrichtung über der ersten Schicht angeordnet ist. Ferner wäre es möglich, zumindest eine Heizeinrichtung über und zumindest eine Heizeinrichtung unter der ersten Schicht anzuordnen.

Nach einer weiteren bevorzugten Ausführungsform ist unter der ersten Schicht ein Zwischenraum angeordnet, welcher mit einem vorzugsweise gasförmigen Fluid, beispielsweise Luft, ausgefüllt ist. Vorzugsweise ist unter und/oder über diesem Zwischenraum eine Heizeinrichtung vorgesehen. Vorteilhafterweise wird die sich in dem Zwischenraum befindliche Luft durch die Heizeinrichtung erwärmt und kann somit mehr Feuchtigkeit aufnehmen.

Nach einer weiteren bevorzugten Ausführungsform umfasst das zumindest eine Sitzelement zumindest eine weitere erste Schicht, welche unter der ersten Schicht angeordnet ist. Diese zumindest eine weitere erste Schicht besteht ebenso aus einem Material, dass Feuchtigkeit aufnehmen und durchleiten kann. Vorteilhafterweise ist zwischen und/oder unter den ersten Schichten ein Zwischenraum vorgesehen, welche mit einem vorzugsweise gasförmigen Fluid, beispielsweise Luft, ausgefüllt sind. Vorzugsweise ist unter und/oder über diesen Zwischenräumen eine Heizeinrichtung vorgesehen. Vorteilhafterweise wird, die sich in den Zwischenräumen befindliche Luft durch die jeweilige Heizeinrichtung erwärmt und kann somit mehr Feuchtigkeit aufnehmen.

Vorteilhafterweise ist die zumindest eine Heizeinrichtung flächig, bevorzugt vollflächig unter der ersten Schicht angeordnet. Hierdurch kann ein effektives Aufheizen der ersten Schicht erfolgen.

Nach einer weiteren Ausführungsform ist in Höhenrichtung Z, Z' des zumindest einen Sitzelements unter der ersten Schicht ein Abstandsgewirke angeordnet. Der Begriff unter der ersten Schicht kann zum einen bedeuten, dass das Abstandsgewirke sich direkt unter der ersten Schicht befindet. Es kann jedoch auch möglich sein, dass sich noch weitere Schichten zwischen der ersten Schicht und dem Abstandsgewirke befinden. Ein solches Abstandsgewirke hat eine hohe Luftdurchlässigkeit und weist gute Polstereigenschaften auf.

Vorzugsweise ist zwischen der ersten Schicht und dem Abstandsgewirke zumindest eine Heizeinrichtung angeordnet. Demnach ist in Höhenrichtung Z, Z' des Sitzelements unter zumindest einer Heizeinrichtung ein Abstandsgewirke angeordnet.

Vorteilhafterweise ist in Höhenrichtung Z, Z' des zumindest einen Sitzelements unter dem Abstandsgewirke zumindest abschnittsweise ein Formelement angeordnet. Das Formelement besteht aus einem Material, welches einen entsprechenden Widerstand beziehungsweise eine Polsterung hinsichtlich der Druckbelastung durch den Kontakt mit dem Nutzer zur Verfügung stellt. Ferner gibt die förmliche Ausgestaltung des Formelements im Wesentlichen die Form des Sitzelements vor. Das Formelement kann bevorzugt aus einem Kaltschaum bestehen. Es wäre jedoch auch denkbar, wenn das Formelement aus einem anderen formgebenden geeigneten Material besteht. Vorzugsweise weist das Formelement einen Lüfterbereich auf, in welchem eine Vielzahl an Lüftungskanälen vorgesehen ist. Des Weiteren ist es von Vorteil, dass das Formelement einen den Lüfterbereich umrandenden Bereich aufweist. Dieser umrandende Bereich kann Seitenwangen aufweisen. In einer bevorzugten Ausführungsform ist eine entsprechende Steuerung/Regelung des Sitzklimas in diesem Bereich nicht vorgesehen, da der Nutzer in der Regel nur geringen Kontakt mit solchen Seitenwangen hat.

Bevorzugt sind die Lüftungskanäle des Lüfterbereichs mit der zumindest einen Belüftungseinrichtung verbunden. Sind zwei Sitzelemente in Form eines unteren Sitzteils und eines Rückenlehnenelementes vorgesehen, kann wie bereits beschrieben eine aktive Steuerung/Regelung des Sitzklimas von lediglich des unteren Sitzteils oder lediglich des Rückenlehnenelementes vorgesehen sein. Es kann jedoch auch eine aktive Steuerung/Regelung des Sitzklimas von sowohl des unteren Sitzteils als auch des Rückenlehnenelementes vorgesehen sein. Hierzu kann vorteilhafterweise den beiden Sitzelementen jeweils eine Belüftungseinrichtung zugordnet sein, welche auch individuell, entsprechend der Sensordaten ansteuerbar sind. Es wäre jedoch auch denkbar, dass die Lüftungskanäle mit nur einer Belüftungseinrichtung verbunden sind. Eine individuelle Entlüftung der beiden Sitzelemente könnte durch eine vorteilhafte Verriegelungseinrichtung erfolgen, welche in den jeweiligen Verbindungskanälen zwischen der Belüftungseinrichtung und dem jeweiligen Sitzelement angeordnet sind. Mit Hilfe dieser Verriegelungseinrichtung kann bestimmt werden, welches Sitzelement belüftet wird. Gegebenenfalls können auch beide Sitzelemente gleichzeitig belüftet werden.

Das Sitzelement kann vorzugsweise auch noch weitere Schichten aufweisen, welche unter, über oder zwischen den bereits genannten Komponenten sich befinden. Diese Schichten können verschieden ausgeprägt sein und auch verschiedene Eigenschaften besitzen. Ebenso ist es möglich, dass der beschriebene Sitzaufbau durch die beschriebenen Komponenten sich in Lage und Position anders zusammensetzen können.

Der oben beschriebene Aufbau des Sitzelements kann für das untere Sitzteil als auch für das Rückenlehnenelement zutreffen.

Erfindungsgemäß ist das zumindest eine Sitzelement durch zumindest eine Heizeinrichtung aufheizbar. In der ersten Schicht des Sitzelements ist erfindungsgemäß Feuchtigkeit aufgenommen. Ferner ist vorzugsweise Luft in der ersten Schicht eingeschlossen. Durch ein Aufheizen geht, in Abhängigkeit von der Temperatur zumindest ein Teil dieser Feuchtigkeit in den gasförmigen Zustand über. Darüber hinaus wird die sich in dem Sitzelement befindliche Luft erwärmt, welche somit mehr Feuchtigkeit aufnehmen kann. Dementsprechend erhöht sich vorteilhafterweise der Dampfdruck in dem Sitzelement. In dem Sitzelement wird somit ein Dampf erzeugt. Ein solcher Dampf kann im Idealfall eine reine gasförmige Phase sein oder aber auch ein Gemisch aus flüssigen und gasförmigen Bestandteilen.

Durch die Aggregatszustandsveränderung kann sich, aufgrund der aufzubringenden Verdampfungswärme, ein weiterer Vorteil in Form einer erhöhten Kühlung der Oberfläche des Sitzelements ergeben.

Erfindungsgemäß wird der durch das Aufheizen erzeugte Dampf mittels der Belüftungseinrichtung aus dem Sitzelement befördert. Erfindungsgemäß arbeitet die Belüftungseinrichtung in einem Saugbetrieb. Der Dampf wird demnach erfindungsgemäß durch die Belüftungseinrichtung aus dem Sitzelement gesogen. Da durch das Aufheizen des Sitzelements der Dampf eine höhere Temperatur aufweist als die entsprechend umliegenden Luftvolumen, entsteht ein Differenzdruck, welcher eine erhöhte Strömungsgeschwindigkeit des Dampfes verursachen kann. Durch diese erhöhte Luftgeschwindigkeit aufgrund der Thermik und dem Saugbetrieb der Belüftungseinrichtung kann ein schnelles Entfernen des Dampfes, beziehungsweise der Feuchtigkeit aus dem Sitzelement erfolgen.

Es wäre jedoch auch denkbar, dass sich die Belüftungseinrichtung in einem Blasbetrieb befindet. Es kann somit Luft in die erste Schicht eingebracht werden, wodurch der Dampf aus der ersten Schicht gedrängt, beziehungsweise aus der ersten Schicht entfernt wird.

Nach eine bevorzugten Ausführungsform umfasst die Sensoreinrichtung einen Sitzbelegungssensor, welcher den Komfortparameter Sitzbelegung detektiert. Durch die vorteilhafte Detektion der Sitzbelegung können bestimmte bevorzugte Modi, welche erfordern, dass keine Sitzbelegung vorliegt, automatisch durchgeführt werden. Der Sitzbelegungssensor kann an einer beliebigen geeigneten Position in dem zumindest einem Sitzelement angeordnet sein.

Nach einer weiteren bevorzugten Ausführungsform erfolgt die Regelung des Sitzklimas durch abwechselndes Heizen und Belüften der ersten Schicht. Vorteilhafterweise sind die zumindest eine Heizeinrichtung und/oder die zumindest eine Belüftungseinrichtung in vorgegebenen Intervallen ein-, beziehungsweise ausschaltbar. Vorteilhafterweise ist die zumindest eine Heizeinrichtung derart in Intervallen schaltbar, dass an der Oberfläche des Sitzelements im Wesentlichen keine Temperaturänderung erfolgt. Der Begriff "im Wesentlichen keine Temperaturänderung" ist dahingehend auszulegen, dass eine Temperaturänderung in einem für den Nutzer nicht merklichen Bereich liegt. Eine solche Temperaturänderung kann demnach bevorzugt in einem Bereich von 0°C bis 10°C, weiter bevorzugt von 0°C bis 5°C, weiter bevorzugt von 0°C bis 2,5°C, weiter bevorzugt von 0°C bis 1°C liegen. Die Heizintervalle sind demnach derart kurz ausgelegt, dass zwar eine entsprechende Umwandlung der in dem Sitzelement, beziehungsweise der ersten Schicht, sich befindenden Feuchtigkeit erfolgen kann, die Erwärmung jedoch nicht so lange erfolgt, dass die Oberfläche des Sitzelements, welche mit dem Nutzer in Kontakt ist, sich für den Nutzer merklich erwärmt. Somit kann die in Dampf umgewandelte Feuchtigkeit durch die Belüftungseinrichtung effektiv entfernt werden, ohne dass der Sitz sich für den Nutzer merklich erwärmt.

Vorzugsweise werden die zumindest eine Heizeinrichtung und/oder die zumindest eine Belüftungseinrichtung in vorgegebenen Intervallen ein-, beziehungsweise ausgeschaltet. Vorteilhafterweise wird die zumindest eine Heizeinrichtung derart in Intervallen eingeschaltet, dass eine Aufwärmung des Sitzes beziehungsweise des Sitzelements nicht bis zur Oberfläche des Sitzelements dringt.

Üblicherweise ist die Wärmeausbreitung umgekehrt proportional zu der Ausbreitungslänge, welche vorliegend der Höhe der ersten Schicht ist. Eine Intervalllänge kann demnach derart bemessen sein, dass auf der Oberfläche des Sitzelements keine wesentliche Temperaturänderung stattfindet, welche von dem Nutzer wahrgenommen wird. Der Mensch erfasst Wärme sehr spät, d. h. die Oberflächentemperatur kann kurzfristig überschritten werden, ohne dass der Nutzer diese wahrnimmt. Durch eine derartige Ausgestaltung kann der Sitz vorzugsweise während der Sitzbelegung klimatisiert werden.

Eine Wärmeleitung bestimmt sich vorteilhafterweise aus dem Wärmekoeffizienten der ersten Schicht (λ), der Temperaturdifferenz (T) zwischen der Heizeinrichtung und der Oberfläche der ersten Schicht und der Höhe (h) wie folgt:

$$\lambda * T * 1/h.$$

Es kann vorteilhaft sein, wenn die Belüftungseinrichtung gleichzeitig kontinuierlich betrieben wird. Alternativ kann die Belüftungseinrichtung abwechselnd mit der zumindest einen Heizeinrichtung betrieben werden.

Das zumindest eine Sitzelement kann demnach in einem Komfortbereich gehalten werden. Ein solcher Komfortbereich der Temperatur liegt vorzugsweise zwischen 28°C und 38°C, idealerweise bei 32 ±5°C. Ein Komfortbereich der Feuchtigkeit liegt vorzugsweise zwischen 10% und 85%, idealerweise bei 50% relativer Luftfeuchtigkeit.

Die zumindest eine Heizeinrichtung und die zumindest eine Belüftungseinrichtung werden vorzugsweise in Abhängigkeit von den kontinuierlich detektierten Komfortparametern betrieben. Durch ein vorteilhaftes Aktivieren der Heizeinrichtung und/oder der Belüftungseinrichtung können die Komfortparameter in den Komfortbereich überführt werden. Sind beispielsweise die detektierten relativen Feuchtigkeitswerte über einem Komfortbereich, kann diese Feuchtigkeit der ersten Schicht und auch der Kleidung des Fahrers effektiv weggeführt werden.

Nach einer weiteren Ausführungsform kann durch abwechselndes Heizen und Belüften der ersten Schicht der Komfortbereich der Komfortparameter eingestellt werden. Insbesondere wenn der Sitz nicht belegt ist, kann hierdurch der Komfortbereich schnell erreicht werden. Durch das vorteilhafte Heizen entsteht wiederum ein Dampf, beziehungsweise ein Dampfdruck in dem zumindest einem Sitzelement. Durch ein vorteilhaftes abwechselndes Zuschalten der Belüftungseinrichtung kann dieser Dampf in dem zumindest einen Sitzelement verteilt und rasch abgesogen werden.

Aufgrund der fehlende Sitzbelegung kann die erste Schicht zu einer höheren Temperatur erhitzt werden, wodurch mehr Flüssigkeit entsprechend in die Gasphase übergeht, beziehungsweise die in dem Fahrzeugsitz befindliche Luft noch mehr Flüssigkeit aufnehmen kann. Das zumindest eine Sitzelement kann somit während einer Belegungspause schnell wieder in den Komfortbereich gebracht werden.

Vorteilhafterweise weist der Fahrzeugsitz hierzu eine Sitzbelegungserkennung auf. Alternativ kann auch eine manuelle Einstellvorrichtung vorgesehen sein, mittels welcher die verschiedenen Modi auswählbar sind.

Durch ein vorteilhaftes abwechselndes Heizen und Belüften der ersten Schicht kann eine Selbstreinigung beziehungsweise eine Desinfektion dieser ersten Schicht stattfinden. Durch das vorteilhafte Entziehen der Feuchtigkeit aus der ersten Schicht wird Bakterien der Nährboden entzogen. Das Entfeuchten erfolgt vorteilhafterweise durch gezieltes Erhitzen und Ablüften der ersten Schicht.

Nach einem weiteren Gedanken der Erfindung erfolgt eine antibakterielle Reinigung beziehungsweise Desinfektion des Sitzelements durch eine Erhöhung der Sitztemperatur auf eine Temperatur, bei welcher Bakterien und Keime nicht überleben können. Vorzugsweise wird die Temperatur auf über 80°C erhöht. Dadurch können, sich in dem zumindest einem Sitzelement befindliche Bakterien und Keime, effektiv abgetötet werden. Vorteilhafterweise erfolgt dies ohne Sitzbelegung. Hierzu ist keine Detektion der Komfortparameter Temperatur und Feuchtigkeit mittels der Komfortparametersensoren notwendig.

Vorzugsweise kann eine Vorkonditionierung des Sitzelements stattfinden. Dies bedeutet ein Einstellen der Komfortparameter vor der Sitzbelegung. Vorzugsweise startet die Vorkonditionierung nach einem vorgebbaren Startereignis. Ein solches Startereignis kann beispielsweise ein Erreichen einer Zeitvorgabe sein. Demzufolge würde die Regel-/Steuereinrichtung ein Zeitelement, eine Uhr oder Ähnliches umfassen oder mit einem solchen Element signaltechnisch verbunden sein. Anschließend regelt, beziehungsweise steuert die Regel-/Steuereinrichtung die Komfortparameter des Sitzelements in den Komfortbereich. Ein solches Startereignis kann beispielsweise das Entsperren des Fahrzeugs sein, eine Annäherung des Nutzers an das Fahrzeug, welches durch einen entsprechenden Sensor detektiert wird.

In den oben beschriebenen Ausführungsformen kann jeweils ein Sitzelement oder mehrere Sitzelemente, beispielsweise ein unteres Sitzteil und ein Rückenlehnenelement entsprechend betrieben werden.

Die Aufgabe wird weiterhin gelöst durch ein Verfahren zum Betrieb eines Fahrzeugsitzes. Ein solches Verfahren zum Betrieb eines Fahrzeugsitzes, welcher zumindest ein Sitzelement, in welchem eine Heizeinrichtung und eine Belüftungseinrichtung vorgesehen ist, umfasst folgende Verfahrensschritte:

a) Erfassen von Sensordaten durch die Sensoreinrichtung welche zumindest einen Temperatursensor und zumindest einen Feuchtigkeitssensor aufweist;

b) Optional vergleichen der erfassten Sensordaten mit vorgegebenen Sollwerten durch die Regel-/Steuereinrichtung;

c) Aktivieren zumindest einer Heizeinrichtung und/oder zumindest einer Belüftungseinrichtung durch die Regel-/Steuereinrichtung, wobei das zumindest eine Sitzelement eine erste Schicht umfasst, welche aus einem Material besteht, das Feuchtigkeit aufnehmen und durchleiten kann sowie das Material der ersten Schicht porös und/oder offenporig ausgebildet ist, wobei das zumindest eine Sitzelement durch zumindest eine Heizeinrichtung aufheizbar ist, wodurch eine in der ersten Schicht aufgenommene Feuchtigkeit zumindest in einen gasförmigen Zustand übergeht und sich die in dem Sitzelement befindliche Luft erwärmt, wobei der durch das Aufheizen erzeugte Dampf mittels der Belüftungseinrichtung aus dem Sitzelement beförderbar ist, wobei der Dampf mittels der Belüftungseinrichtung aus dem Sitzelement gesogen wird.

Das Verfahren kann dabei mit allen bereits obig im Rahmen der Vorrichtung beschriebenen Merkmalen einzeln oder in Kombination miteinander ausgestattet sein und umgekehrt. Bevorzugt ist die Vorrichtung dazu geeignet und bestimmt, die beschriebenen Ausführungsformen des Verfahrens (insbesondere einzelne und/oder mehrere) auszuführen.

Bei dem Verfahren sind vorteilhafterweise verschiedene Betriebsmodi einstellbar. Dies kann vorteilhafterweise durch eine geeignete Eingabeeinrichtung erfolgen. Die Verfahren unterscheiden sich dabei vorteilhafterweise darin, ob der Sitz belegt ist oder nicht. Vorteilhafterweise umfasst demnach der Schritt a) ebenso das Erfassen von Sensordaten eines Sitzbelegungssensors.

Nach einer bevorzugten Ausführungsform wird bei einer nicht vorhandenen Sitzbelegung durch die Regel-/Steuereinrichtung (9) eine antibakterielle Reinigung des Sitzelements ausgeführt, in dem die zumindest eine Heizeinrichtung (10) angesteuert wird, so dass das zumindest eine Sitzelement auf eine Temperatur gebracht wird, bei welcher Bakterien und Keime abgetötet werden, wobei diese Temperatur 80°C beträgt.

Vorzugsweise wird bei der antibakteriellen Reinigung gleichzeitig die Belüftungseinrichtung betrieben. Alternativ können die Heizeinrichtung und die Belüftungseinrichtung abwechselnd betrieben werden. Durch das vorteilhafte Entziehen der Feuchtigkeit aus dem zumindest einem Sitzelement, beziehungsweise aus der ersten Schicht des zumindest einen Sitzelements, wird Bakterien der Nährboden entzogen. Das Entfeuchten erfolgt vorteilhafterweise durch gezieltes Erhitzen und Ablüften der ersten Schicht. Durch ein vorteilhaftes abwechselndes Heizen und Belüften der ersten Schicht kann eine Selbstreinigung beziehungsweise eine Desinfektion dieser ersten Schicht stattfinden.

Nach einer bevorzugten Ausführungsform werden bei einer vorhandenen Sitzbelegung durch die Regel-/Steuereinrichtung die zumindest eine Heizeinrichtung und die zumindest eine Belüftungseinrichtung in abwechselnden Intervallen aktiviert. Vorteilhafterweise ist die Intervalllänge für eine Aktivierung der zumindest einen Heizeinrichtung derart ausgelegt, dass an der Oberfläche des Sitzelements im Wesentlichen keine Temperaturänderung erfolgt. Der Begriff "im Wesentlichen keine Temperaturänderung" ist dahingehend auszulegen, dass eine Temperaturänderung in einem für den Nutzer nicht merklichen Bereich liegt. Eine solche Temperaturänderung kann demnach bevorzugt in einem Bereich von 0°C bis 10°C, weiter bevorzugt von 0°C bis 5°C, weiter bevorzugt von 0°C bis 2,5°C, weiter bevorzugt von 0°C bis 1°C liegen. Die in die erste Schicht eingebrachte Wärmemenge ist jedoch ausreichend, um eine ausreichende Menge an Feuchtigkeit in Dampf umzuwandeln. Der erzeugte Dampf kann bevorzugt aus einer Gasphase und gegebenenfalls aus einer Flüssigkeitsphase bestehen.

Das vorteilhafte Verfahren der in Intervallen abwechselnden Aktivierung der Heizeinrichtung und der Belüftungseinrichtung ist dann vorteilhaft anzuwenden, wenn der feuchtigkeitswert oberhalb der Komfortgrenze liegt. Gleichzeitig liegt der Temperaturwert jedoch innerhalb oder über den Komfortgrenzen. Durch diesen Modus kann sowohl dem Sitzelement als auch der Kleidung des Nutzers Feuchtigkeit entzogen werden, wodurch diese trocknen.

Befindet sich die Temperatur unterhalb des Komfortbereichs, kann die Intervalllänge demensprechend angepasst werden, dass eine ausreichende Wärmemenge in das Sitzelement, beziehungsweise die erste Schicht des Sitzelement eingeführt wird, dass deren Temperatur wieder in den Komfortbereich überführt werden.

Ein solcher Komfortbereich der Temperatur liegt vorzugsweise zwischen 28°C und 38°C, idealerweise bei 32±5°C. Ein Komfortbereich der Feuchtigkeit liegt vorzugsweise zwischen 10% und 85%, idealerweise bei 50% relativer Luftfeuchtigkeit.

Nach einer bevorzugten Ausführungsform werden bei einer nicht vorhandenen Sitzbelegung durch die Regel-/Steuereinrichtung, die zumindest eine Heizeinrichtung und die zumindest eine Belüftungseinrichtung in abwechselnden Intervallen aktiviert. Vorteilhafterweise ist die Intervalllänge für eine Aktivierung der zumindest einen Heizeinrichtung derart ausgelegt, dass das zumindest eine Sitzelement auf eine Temperatur erwärmt wird, welche über einem vorgegebenen Komfortbereich liegt. Vorzugsweise liegt der Komfortbereich der Temperatur zwischen 28°C und 38°C. Weiter bevorzugt liegt der Komfortbereich der Temperatur bei 32±5°C. Demnach erfolgt in diesem Modus eine Temperaturänderung an der Oberfläche des Sitzelements, welche durch die Nichtbelegung des Sitzes jedoch belanglos ist. Durch die Verwendung einer höheren Temperatur geht mehr Flüssigkeit entsprechend in die Gasphase über, beziehungsweise die in dem Fahrzeugsitz befindliche Luft wird noch mehr Flüssigkeit aufnehmen können. Das zumindest eine Sitzelement kann somit während einer Belegungspause schneller wieder in den Komfortbereich gebracht werden.

Nach einer weiteren bevorzugten Ausführungsform erfolgt bei Eintritt eines vorgegeben Startereignis eine Vorkonditionierung des zumindest einen Sitzelements, in welcher durch Aktivierung der zumindest einen Heizeinrichtung und/oder der zumindest einen Belüftungseinrichtung durch die Regel-/Steuereinrichtung die Komfortparameter in den vorgegebenen Komfortbereich gebracht werden. Dies bedeutet ein Einstellen der Komfortparameter vor der Sitzbelegung. Vorzugsweise startet die Vorkonditionierung nach einem vorgebbaren Startereignis. Ein solches Startereignis kann beispielsweise ein Erreichen einer Zeitvorgabe sein. Demzufolge würde die Regel-/Steuereinrichtung ein Zeitelement, eine Uhr oder Ähnliches umfassen oder mit einem solchen Element signaltechnisch verbunden sein. Anschließend regelt beziehungsweise steuert die Regel-/Steuereinrichtung die Komfortparameter des Sitzelements in dem Komfortbereich. Ein solches Startereignis kann beispielsweise das Entsperren des Fahrzeugs sein, eine Annäherung des Nutzers an das Fahrzeug, welches durch einen entsprechenden Sensor detektiert wird.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:

Fig.1a    ein Sitzelement gemäß einer Ausführungsform der Erfindung;

Fig.1b    ein Sitzelement gemäß einer Ausführungsform der Erfindung;

Fig.1c    ein Sitzelement gemäß einer Ausführungsform der Erfindung;

Fig.2    ein Sitzelement gemäß einer Ausführungsform der Erfindung;

Fig.3    Diagramm zum Komfortbereich des Nutzers;

Fig.4    Ablaufdiagramm bei Sitzbelegung;

Fig.5    Ablaufdiagramm bei Sitzbelegung;

Fig.6    Ablaufdiagramm eines Verfahrens zum Betreiben eines Sitzes in der Gesamtübersicht;

Fig.7    Ablaufdiagramm eines Verfahrens zum Betreiben eines Sitzes in einer Unteransicht;

Fig.8    Ablaufdiagramm eines Verfahrens zum Betreiben eines Sitzes in einer Unteransicht;

Fig.9    Ablaufdiagramm eines Verfahrens zum Betreiben eines Sitzes in einer Unteransicht;

Fig.10    Ablaufdiagramm eines Verfahrens zum Betreiben eines Sitzes in einer Unteransicht;

Fig 11    Schematische Darstellung der verschiedenen Fälle bei einem Verfahren zum Betreiben eines Sitzes;

Fig 12    Schematische Darstellung der verschiedenen Fälle bei einem Verfahren zum Betreiben eines Sitzes.

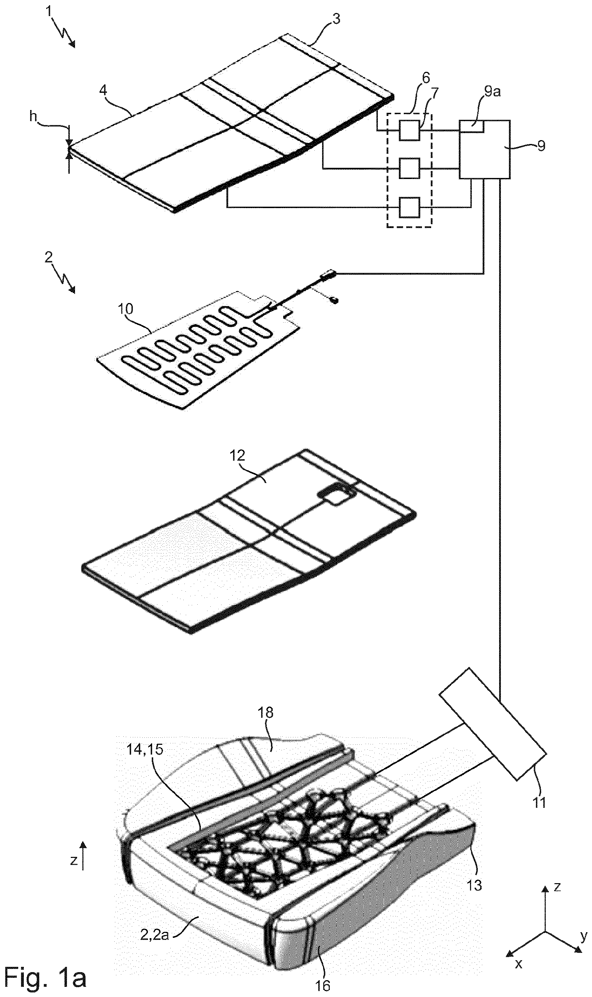

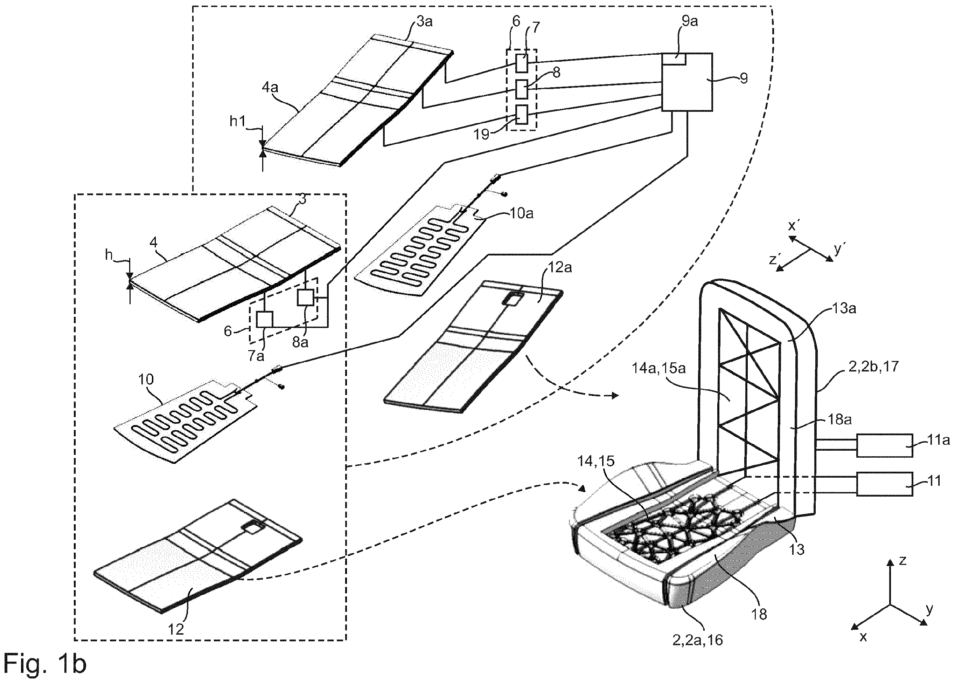

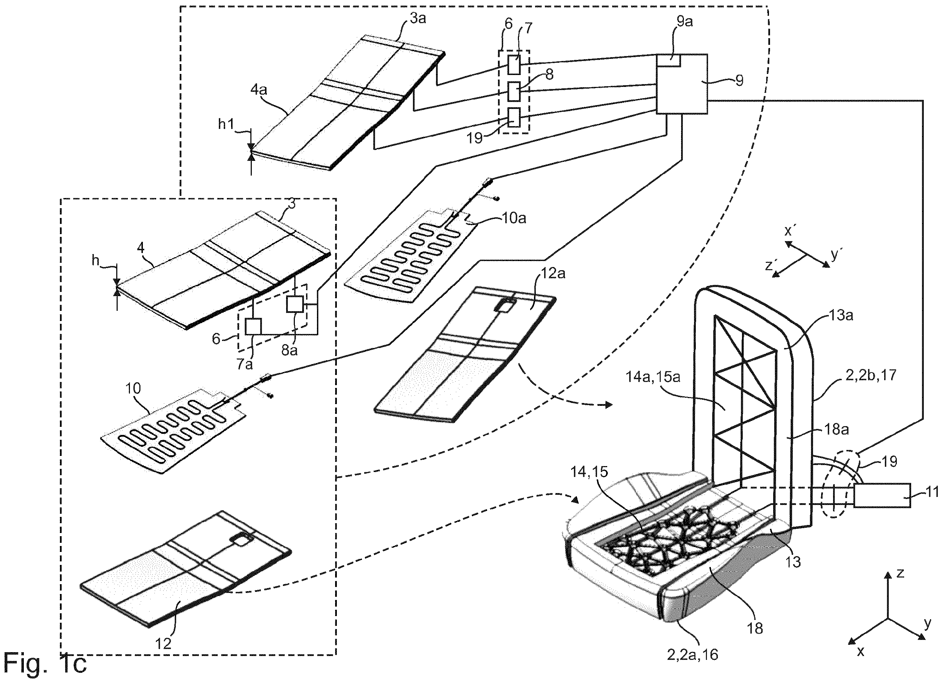

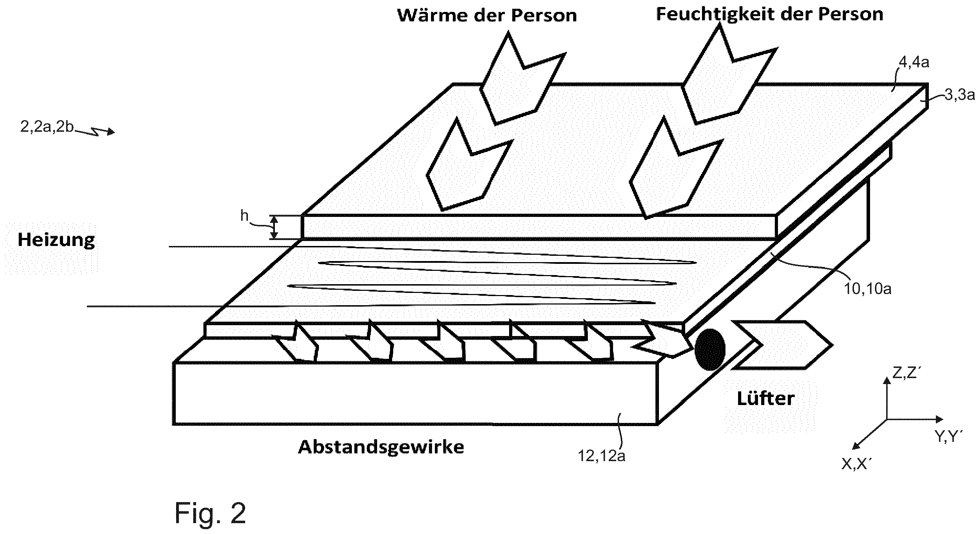

In den Figuren 1a, 1b, 1c und 2 ist der Aufbau eines Sitzes (1) beziehungsweise eines Sitzelementes (2) dargestellt. Ein solcher Fahrzeugsitz (1) umfasst zumindest ein Sitzelement, in welchem zumindest eine Heizeinrichtung (10) und zumindest eine Belüftungseinrichtung (11) vorgesehen ist, wobei der Fahrzeugsitz (1) eine Sensoreinrichtung (6) umfasst, welche zumindest einen Temperatursensor (7) und zumindest einen Feuchtigkeitssensor (8) aufweist, wobei eine Regel-/ Steuereinrichtung (9) anhand der Daten der Sensoreinrichtung (6) bezüglich der Komfortparameter, Temperatur und/oder Feuchtigkeitsgehalt, die Heizeinrichtung (10) und/oder die Belüftungseinrichtung (11) steuert oder regelt, wodurch das Sitzklima aktiv steuerbar oder regelbar ist.

Die Regel-/Steuereinrichtung (9) weist eine Speichereinrichtung (9a) auf, in welcher Sollwerte oder Sollbereiche für die Komfortparameter oder Sollwerte oder Sollbereiche für Komfortparameterkombinationen abgelegt sind. Dabei vergleicht die Regel-/Steuereinrichtung (9) die durch die Sensoreinrichtung (6) bereitgestellten Daten mit den entsprechenden Sollwerten oder Sollbereichen.

Der Fahrzeugsitz (1) umfasst zwei Sitzelemente (2, 2a, 2b), wobei ein Sitzelement (2a) ein unteres Sitzteil (16) ist und das weitere Sitzelement (2, 2b) ein Rückenlehnenelement (17) ist. Dies ist in Figur 1b und 1c dargestellt. In Figur 1a ist lediglich ein Sitzelementen (2, 2a, 2b) in Form eines unteren Sitzteils (16) dargestellt.

Die Sensoreinrichtung (6) umfasst zumindest einen Temperatursensor (7) und zumindest einen Feuchtigkeitssensor (8) für das untere Sitzteil (16) und zumindest einen Temperatursensor (7a) und zumindest einen Feuchtigkeitssensor (8a) für das Rückenlehnenelement (17).

Die Sitzelemente (2, 2a, 2b) umfassen jeweils eine Höhenrichtung Z, Z'. Die Höhenrichtung Z, Z' ist derart zu verstehen, dass die oberste Schicht des Sitzelements (2, 2a, 2b) diejenige ist, welche mit dem Nutzer in Kontakt ist und das unterste Element dasjenige ist, welches am weitesten von dem Nutzer entfernt ist. Ferner umfassen die Sitzelemente (2, 2a, 2b) jeweils eine Längsrichtung X, X' und eine Breitenrichtung Y, Y'.

Das zumindest eine Sitzelement (2, 2a, 2b) umfasst eine erste Schicht (3, 3a), welche aus einem Material besteht, das Feuchtigkeit aufnehmen und durchleiten kann, wobei das Material der ersten Schicht (3, 3a) porös und/oder offenporig ausgebildet ist. Eine Oberfläche (4, 4a) der ersten Schicht (3, 3a) ist bei Sitzbelegung in direktem Kontakt mit dem Nutzer oder in unmittelbarer Nähe zu dem Nutzer. Es kann auch vorteilhaft sein, dass diese erste Schicht (3) von einem Bezug, beispielsweise einem Stoffbezug, bedeckt wird.

Die Sensoreinrichtung (6) umfasst zumindest einen Temperatursensor (7) und/oder zumindest einen Feuchtigkeitssensor (8), welche in dem zumindest einem Sitzelement (2, 2a, 2b) derart angeordnet ist, dass die Komfortparameter Temperatur und/oder Feuchtigkeit an oder nahe der Oberfläche (4, 4a) des zumindest einem Sitzelements (2, 2a, 2b) erfassbar sind. Der zumindest eine Temperatursensor (7, 7a) und/oder der zumindest eine Feuchtigkeitssensor (8, 8a) ist in und/oder über und/oder unter der ersten Schicht (3, 3a) angeordnet, wobei die Regel-/Steuereinrichtung (9) die von der Sensoreinrichtung (6) übertragenen Daten in Abhängigkeit zum Abstand des jeweiligen Sensors (7, 7a, 8, 8a) zu der mit dem Nutzer in Kontakt stehenden Oberfläche (4,4a) des zumindest einen Sitzelements (2,2a,2b) ausgewertet werden.

Die Sensoreinrichtung (6), ist mit einer Regel-/Steuereinrichtung (9) signaltechnisch verbunden. Die Regel-/Steuereinrichtung (9) steuert die zumindest eine Heizeinrichtung (10, 10a) und/oder die zumindest eine Belüftungseinrichtung (11, 11a) an.

In den Figuren 1a, 1b und 2 ist ersichtlich, dass in Höhenrichtung (Z, Z') des Sitzelements (2, 2a, 2b) unter der ersten Schicht (3, 3a) zumindest eine Heizrichtung (10, 10a) angeordnet ist. Der Begriff "unter" der ersten Schicht (3, 3a) ist dahingehend zu verstehen, dass zumindest eine Heizeinrichtung (10, 10a) direkt unter der ersten Schicht (3, 3a) angeordnet sein kann oder dass noch weitere Schichten mit verschiedenen Funktionalitäten und Eigenschaften zwischen der ersten Schicht (3, 3a) und der zumindest einen Heizeinrichtung (10, 10a) vorhanden sein können. Vorteilhafterweise ist die zumindest eine Heizeinrichtung (10, 10a) flächig, bevorzugt vollflächig, unter der ersten Schicht (3, 3a) angeordnet.

Nach einer weiteren Ausführungsform ist in Höhenrichtung (Z, Z') des Sitzelements (2, 2a, 2b) unter der der ersten Schicht (3, 3a) ein Abstandsgewirke (12, 12a) angeordnet. Der Begriff "unter" der ersten Schicht (3, 3a) ist wiederum dahingehend zu verstehen, dass das Abstandsgewirke (12, 12a) direkt unter der ersten Schicht (3, 3a) angeordnet sein kann oder dass noch weitere Schichten mit verschiedenen Funktionalitäten und Eigenschaften oder die Heizeinrichtung (10, 10a) zwischen der ersten Schicht (3, 3a) und dem Abstandsgewirke (12, 12a) vorhanden sein können.

Weiterhin ist in Höhenrichtung Z, Z' des Sitzelements (2, 2a, 2b) unter dem Abstandsgewirke (12, 12a) zumindest teilweise ein Formelement (13, 13a) angeordnet. Ein solches Formelement (13, 13a) weist einen Lüfterbereich (14, 14a) auf, in welchem eine Vielzahl an Lüftungskanälen (15, 15a) vorgesehen ist. Das Formelement (13, 13a) weist einen den Lüfterbereich (14, 14a) umrandenden Bereich (18, 18a) auf. Dieser umrandende Bereich (18, 18a) kann sich entlang der Breiterichtung Y, Y' als auch entlang der Längsrichtung X, X' erstrecken. Der sich entlang der Längsrichtung X, X' erstreckende umrandende Bereich (18, 18a) ist als Seitenwangen ausgebildet.

Die Lüftungskanäle (15, 15a) sind mit der Belüftungseinrichtung (11, 11a) verbunden. In der Ausführungsform gemäß Figur 1b ist für das untere Sitzteil (16) und für das Rückenlehnenelement (17) jeweils eine Belüftungseinrichtung (11, 11a) vorgesehen. In der Ausführungsform gemäß Figur 1c sind sowohl das untere Sitzteil (16) als auch das Rückenlehnenelement (17) mit lediglich einer Belüftungseinrichtung (11) verbunden. Hierbei ist vorteilhafterweise eine Verriegelungseinrichtung (19) vorgesehen, mittels welcher die jeweiligen Verbindungskanäle zwischen der Belüftungseinrichtung 11) und dem jeweiligen Sitzelement (2, 2a, 2b) verriegelbar ist. Mit Hilfe diese Verriegelungseinrichtung (20) kann bestimmt werden, welches Sitzelement (2, 2a, 2b) belüftet wird. Gegebenenfalls können auch beide Sitzelemente (2, 2a, 2b) gleichzeitig belüftet werden. Die Verriegelungseinrichtung (20) ist mit der Regel-/Steuereinrichtung (9) signaltechnisch verbunden und wird von dieser angesteuert.

Das Abstandsgewirke (12, 12a) und der Lüfterbereich (14, 14a) sind flächig, bevorzugt vollflächig, unter der Heizeinrichtung (10, 10a), beziehungsweise der ersten Schicht (3, 3a) angeordnet.

Das Sitzelement (2, 2a, 2b) kann auch noch weitere Schichten aufweisen, welche unter, über oder zwischen den genannten Komponenten sich befinden. Diese Schichten können verschieden ausgeprägt sein und auch verschiedene Eigenschaften besitzen. Ebenso ist es möglich, dass der beschriebene Sitzaufbau durch die beschriebenen Komponenten sich in Lage und Position anders zusammensetzen können.

Es können auch zwei oder mehrere Heizeinrichtungen (10, 10a) in dem Sitzelement (2, 2a, 2b) vorgesehen sein, um einen größeren Abstand zur Oberfläche (4, 4a) zu erreichen, und die im Folgenden beschriebene Boost-Funktion besser nutzen zu können.

Schließlich umfasst die Sensoreinrichtung (6) einen Sitzbelegungssensor (19), welcher die Sitzbelegung detektiert.

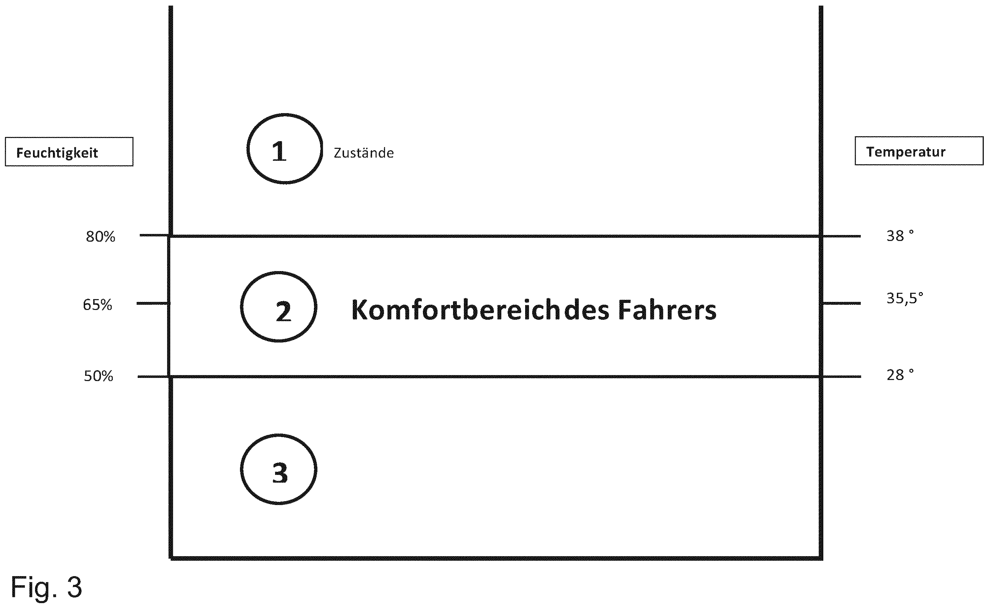

In Figur 3 sind die Komfortparameter und ein entsprechender Komfortbereich eines Sitzelements (2, 2a, 2b) dargestellt. Der Komfortbereich der Temperatur liegt vorteilhafterweise in einem Bereich zwischen 28°C und 38°C. Der Komfortbereich der Feuchtigkeit liegt in einem Bereich zwischen 50% und 80% relative Feuchtigkeit. In einem definierten Zustand 1 liegen die Komfortparameter über dem Komfortbereich des Nutzers. Sind die Komfortparameter in dem Komfortbereich, ist Zustand 2 vorliegend. In einem Zustand 3 liegen die Komfortparameter unter einem Komfortbereich des Nutzers.

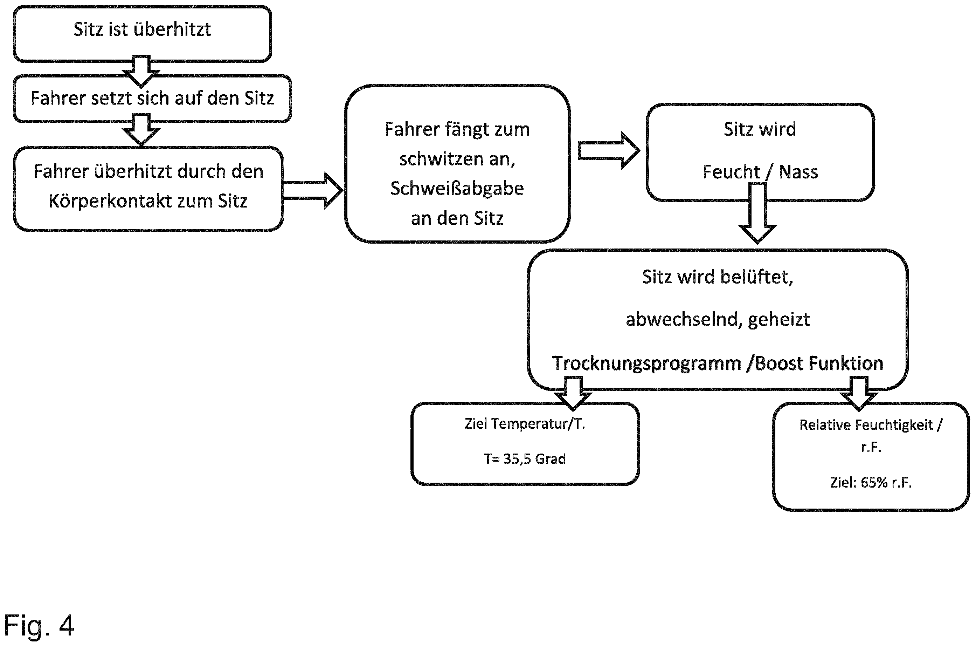

In Figur 4 ist ein möglicher Zustand im Winter dargestellt. Der Sitz ist dabei durch die vorherige manuelle Einstellung der Sitzheizung überhitzt. Der Nutzer überhitzt demnach ebenso durch den Körperkontakt mit dem Sitzelement (2, 2a, 2b). Demensprechend schwitzt der Nutzer. Die Feuchtigkeit wird durch die erste Schicht (3, 3a) des Sitzelements (2, 2a, 2b) aufgenommen.

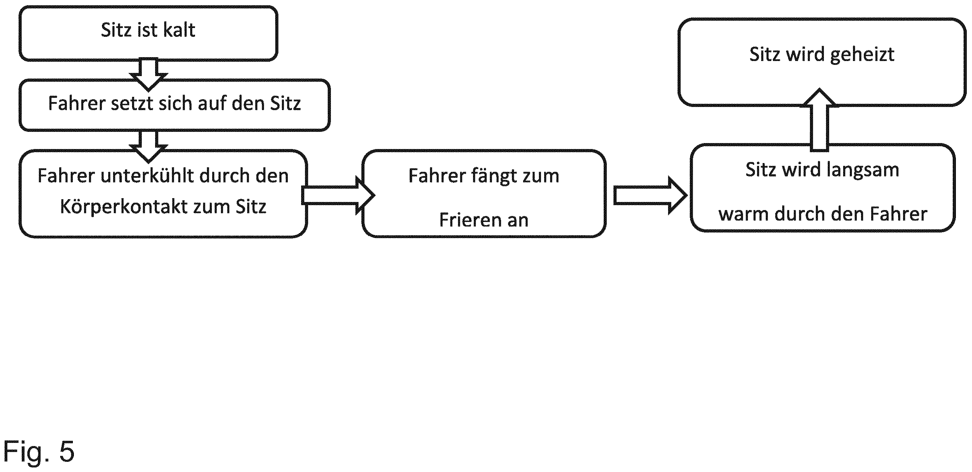

Durch die Boost-Funktion, welche im Folgenden beschrieben wird, kann das Sitzelement (2, 2a, 2b) schnell getrocknet werden. Es können somit eine ideale Temperatur von 35,5 °C und eine ideale relative Feuchtigkeit von 65% eingestellt werden. In dem Szenario nach Figur 5 ist das Sitzelement (2, 2a, 2b) kalt. Der Fahrer unterkühlt durch den Körperkontakt mit dem Sitzelement (2, 2a, 2b), da dieser Körperwärme an den Sitz (1) abgibt. Durch das Heizelement (10, 10a) kann das Sitzelement entsprechend aufgeheizt werden.

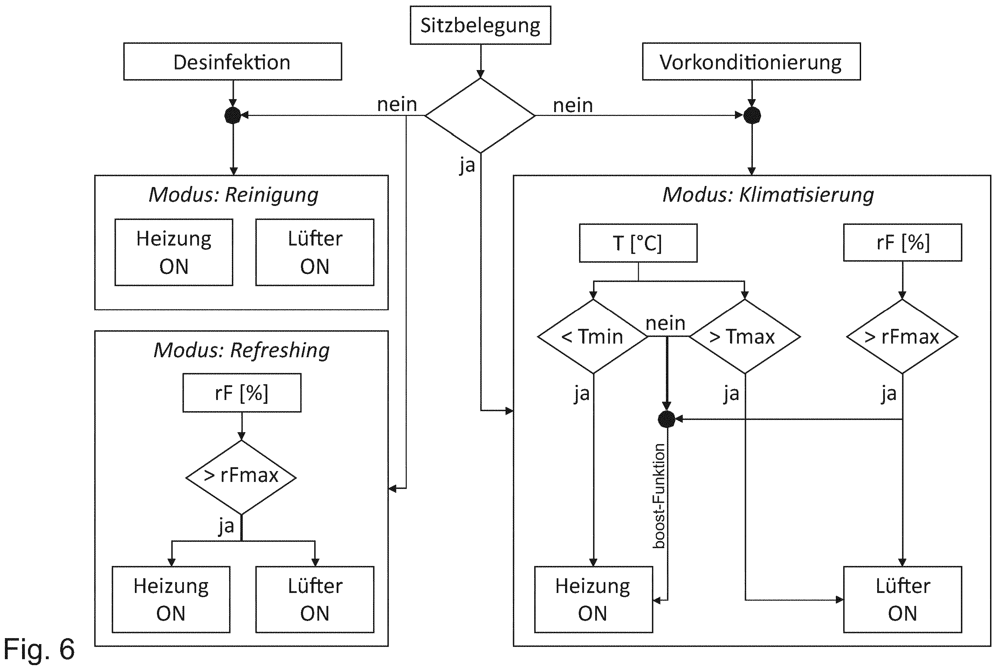

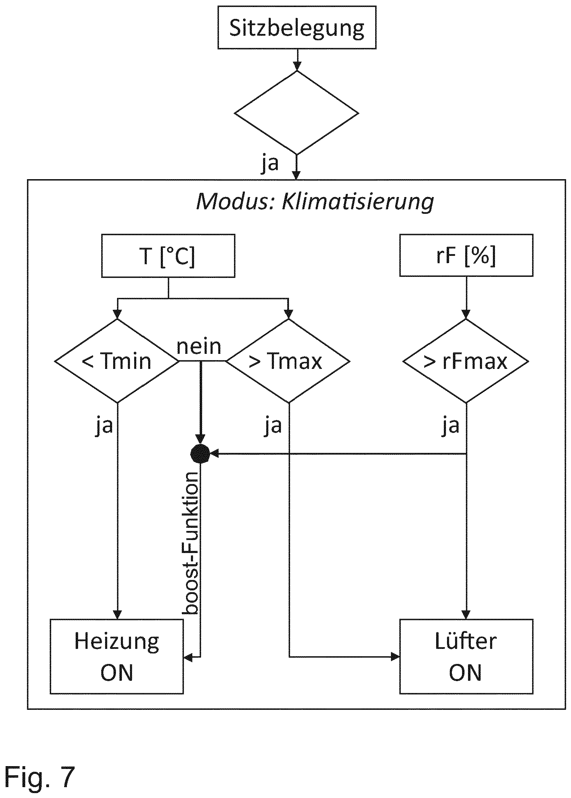

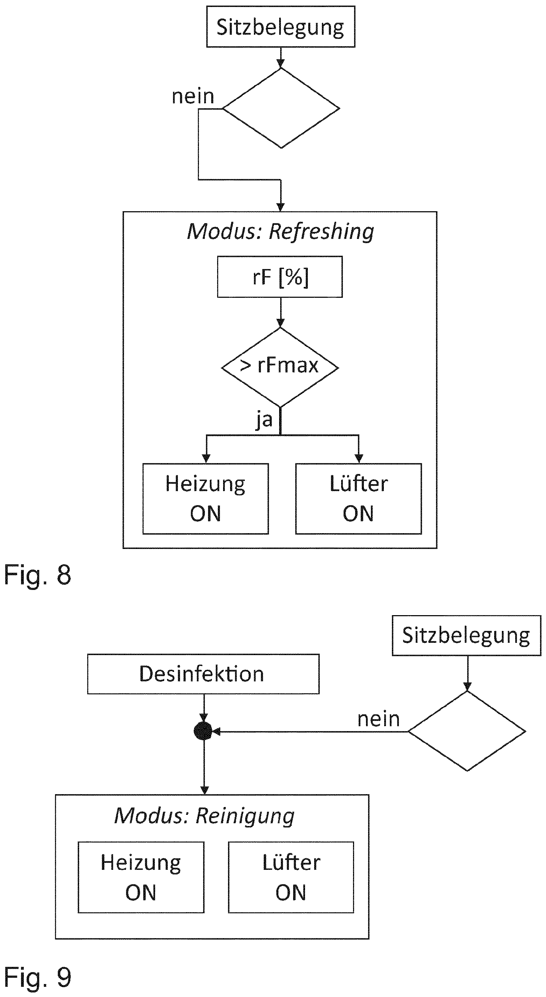

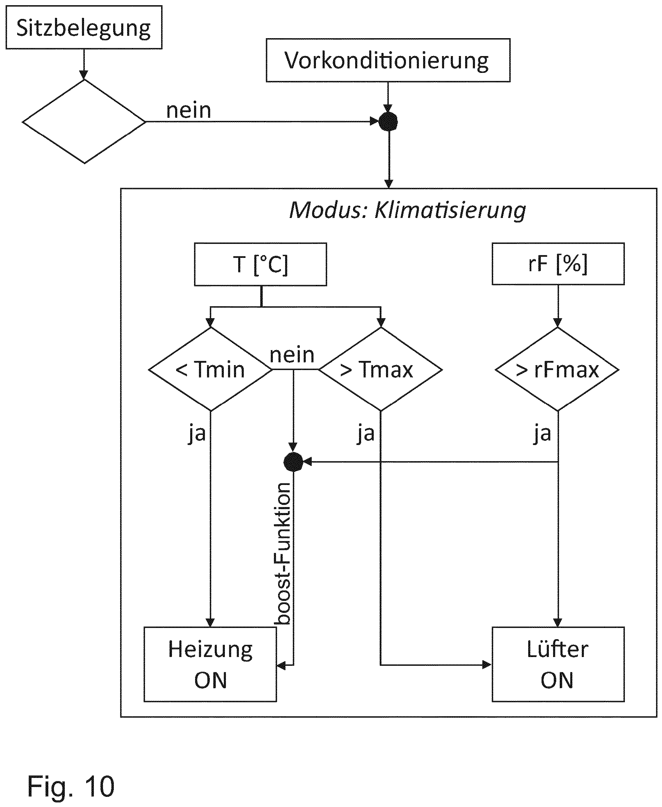

In Figur 6 ist ein entsprechendes Ablaufdiagramm für ein Verfahren zum bevorzugten Betreiben eines Sitzes (1) in einer Gesamtübersicht gezeigt. Die Figuren 7 bis 10 zeigen die entsprechenden Unterübersichten. Das Verfahren bietet vorzugsweise folgenden Modi an: Klimatisierung mit Boost-Funktion (Figur 7), Refreshing (Figur 8), Reinigung (Figur 9), Desinfektion (Figur 9) und Klimatisierung mit Vorkondition (Figur 10).

Eine vorteilhafte Reinigung des Sitzes (1) kann durch ein Aktivieren der Heizeinrichtung (10, 10a) und der Belüftungseinrichtung (11, 11a) erfolgen. Eine Reinigung des Sitzes (1) kann bevorzugt erfolgen, nachdem keine Sitzbelegung detektiert wurde. Hierzu wird die erste Schicht (3, 3a) durch die Heizeinrichtung (10, 10a) vorteilhafterweise erhitzt. Durch das Aufheizen geht, in Abhängigkeit von der Temperatur, zumindest ein Teil der in der ersten Schicht (3, 3a) enthaltenen Feuchtigkeit in den gasförmigen Zustand über. Darüber hinaus wird die sich in der ersten Schicht (3, 3a) befindliche Luft erwärmt, welche somit mehr Feuchtigkeit aufnehmen kann. Es wird somit in der ersten Schicht (3, 3a) ein Dampf erzeugt. Ein solcher Dampf kann im Idealfall eine reine gasförmige Phase sein oder aber auch ein Gemisch aus flüssigen und gasförmigen Bestandteilen. Durch die Belüftungseinrichtung (11, 11a) wird der Dampf bevorzugt durch das Abstandsgewirke (12, 12a) und den Lüfterbereich (14, 14a) beziehungsweise die Lüftungskanäle (15, 15a) aus dem Sitzelement (2, 2a, 2b) abgesaugt. Alternativ kann in dem Modus "Desinfektion" die erste Schicht (3, 3a) auf eine Temperatur über 80°C erhitzt werden. Dadurch können vorhandene Bakterien effektiv abgetötet werden.

In dem bevorzugten Modus "Refreshing" (Figur 8) wird durch den Feuchtigkeitssensor (8) eine relative Feuchtigkeit in der ersten Schicht (3, 3a) detektiert, welche über dem Komfortbereich liegt. Durch vorteilhaftes abwechselndes Aktivieren der Heizeinrichtung (10, 10a) und der Belüftungseinrichtung (11, 11a) durch die Regel-/Steuereinrichtung (9) kann die relative Feuchtigkeit schnell wieder zu einem Wert in dem Komfortbereich geregelt werden. Durch das Heizen entsteht wiederum ein Dampf, beziehungsweise ein Dampfdruck in dem Sitzelement (2, 2a, 2b). Durch abwechselndes Zuschalten der Belüftungseinrichtung (11, 11a) kann dieser Dampf in der ersten Schicht (3, 3a) verteilt und rasch abgesogen werden. Der "Refreshing"- Modus wird vorzugsweise bei fehlender Sitzbelegung durchgeführt. Die erste Schicht (3, 3a) kann hier vorteilhafterweise auf eine höhere Temperatur erhitzt werden, wodurch mehr Flüssigkeit entsprechend in die Gasphase übergeht, beziehungsweise die in der ersten Schicht (3, 3a) befindliche Luft noch mehr Flüssigkeit aufnehmen kann. Das Sitzelement (2, 2a, 2b) kann somit während einer Belegungspause schnell wieder in den Komfortbereich gebracht werden.

Im Falle einer detektierten Sitzbelegung kann eine bevorzugte Klimatisierung des Sitzes (Figur 7) erfolgen. Ferner kann eine solche Klimatisierung nach einer entsprechenden vorteilhaften Vorkonditionierung, welche vor der Sitzbelegung durchgeführt wurde, erfolgen (Figur 8). In dem Modus "Klimatisierung" werden die Komfortparameter Temperatur und relative Feuchtigkeit durch den Temperatursensor (7, 7a) und den Feuchtigkeitssensor (8, 8a) detektiert.

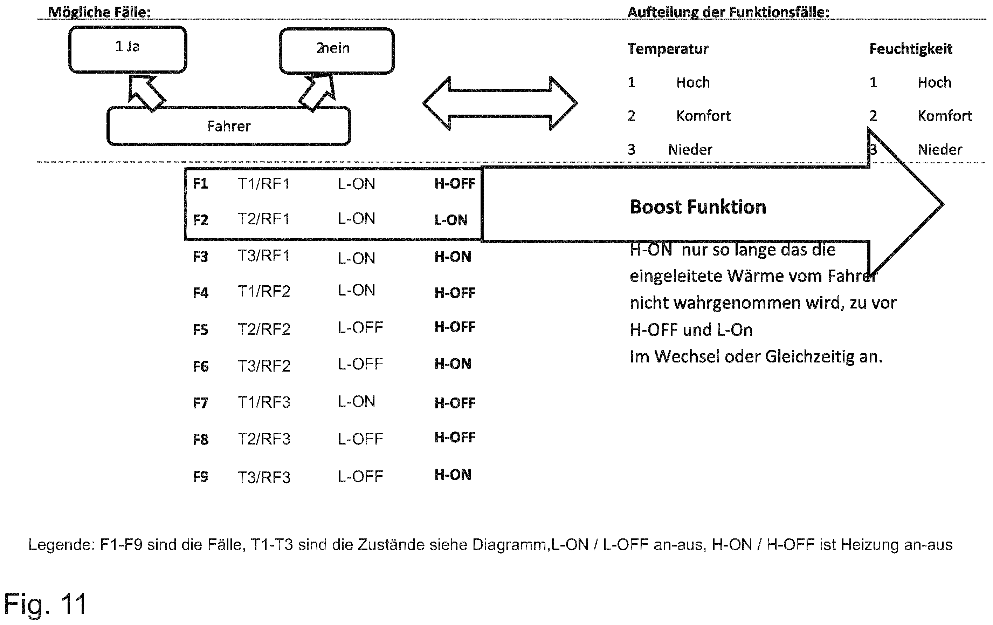

Die entsprechenden bevorzugten Fallkonstellationen (F1 bis F9) sind in Figur 11 gezeigt. So wird bei einer Temperatur unterhalb einer Mindesttemperatur (Tmin) die Heizeinrichtung (10, 10a) aktiviert, bis der vorgegebene Komfortwert erreicht wird.

Die Boost-Funktion wird vorzugsweise aktiviert, wenn die detektierte relative Feuchtigkeit über dem Komfortbereich, beziehungsweise einem maximalen Wert (rFmax) liegt. Gleichzeitig liegt die Temperatur in oder über dem Komfortbereich, also einer maximalen Temperatur (Tmax). Dies sind die Fälle F1 und F2. Durch diese Boost-Funktion soll verhindert werden, dass ein zusätzlicher Temperaturanstieg an der Oberfläche (4, 4a) der ersten Schicht (3, 3a) erfolgt.

Hierzu wird die Heizeinrichtung (10, 10a) bevorzugt in vorgegebenen Intervallen ein-, beziehungsweise ausgeschaltet. Vorteilhafterweise wird die Heizeinrichtung (10, 10a) derart in Intervallen eingeschaltet, dass im Wesentlichen kein Temperaturanstieg der Oberfläche (4, 4a) der ersten Schicht (3, 3a) erfolgt. Üblicherweise ist die Wärmeausbreitung umgekehrt proportional zu der Ausbreitungslänge der Wärme, welche vorliegend in der Höhe (h) der ersten Schicht (3, 3a) ist.

Eine Wärmeleitung bestimmt sich vorteilhafterweise aus dem Wärmekoeffizienten der ersten Schicht (λ), der Temperaturdifferenz (T) zwischen dem Heizelement und der Oberfläche der ersten Schicht und der Höhe (h) wie folgt:

$$\lambda * T * 1/h.$$

Die Intervalllänge wird demnach vorzugsweise derart bemessen, dass bevor eine wesentliche Wärmemenge die Oberfläche (4, 4a) der ersten Schicht (3, 3a) erreicht, die Heizeinrichtung deaktiviert wird. Eine Intervalllänge kann demnach derart bemessen sein, dass auf der Oberfläche des Sitzelements keine wesentliche Temperaturänderung stattfindet, welche von dem Nutzer wahrgenommen wird. Unter einer wesentlichen Temperaturänderung ist eine Änderung um 0°C-10°C, bevorzugt um 0°C-5°C, weiter bevorzugt um 0°C-2,5°C, weiter bevorzugt 0°C bis 1°C zu verstehen. Der Mensch erfasst Wärme sehr spät, d. h. die Oberflächentemperatur kann kurzfristig überschritten werden, ohne dass der Nutzer dies wahrnimmt.

Die in die erste Schicht (3, 3a) eingebrachte Wärmemenge ist jedoch ausreichend, um eine ausreichende Menge an Dampf zu erzeugen, welcher dann durch die Belüftungseinrichtung (11, 11a) abgesaugt werden kann. Durch vorteilhaftes mehrmaliges Wiederholen des Aktivierens des Heizelements kann die Feuchtigkeit aus der ersten Schicht (3, 3a) erhitzt werden. Es kann somit ein Dampf erzeugt werden, welcher aus einer Gasphase und eventuell aus einer Flüssigphase besteht. Dieser Dampf kann dann durch die Belüftungseinrichtung entnommen werden, bis der detektierte Feuchtigkeitswert einem vorbestimmten Wert in dem Komfortbereich entspricht. Während des Aktivierens der Heizeinrichtung (10, 10a) kann die Belüftungseinrichtung (11, 11a) kontinuierlich betrieben werden. Es wäre auch denkbar, dass die Belüftungseinrichtung (11, 11a) im Wechsel mit der Heizeinrichtung (10, 10a) aktiviert, beziehungsweise deaktiviert wird.

Im Fall F3 ist zwar die relative Feuchtigkeit oberhalb des Komfortbereichs, allerdings liegt der detektierte Temperaturwert unter dem minimalen Temperaturwert (Tmin). Demnach ist hier die Boost-Funktion nicht anzuwenden, da es beabsichtigt ist, eine bestimmte Wärmemenge der Oberfläche (4, 4a) der ersten Schicht (3, 3a) zuzuführen.

Im Fall F4 liegt die detektierte Temperatur über dem maximalen Temperaturwert (Tmax). Die detektierte relative Feuchtigkeit liegt jedoch im Komfortbereich. Durch ein Aktivieren der Belüftungseinrichtung (11, 11a) kann die Temperatur entsprechend gesenkt werden. Dementsprechend wird die Heizeinrichtung (10, 10a) nicht aktiviert.

Im Fall F5 liegen sowohl die detektierte Temperatur als auch die detektierte relative Feuchtigkeit im Komfortbereich. Demnach werden weder die der Belüftungseinrichtung (11, 11a) noch die Heizeinrichtung (10, 10a) aktiviert.

Im Fall F6 liegt die detektierte relative Feuchtigkeit im Komfortbereich, die detektierte Temperatur ist jedoch zu niedrig. Demnach wird lediglich die Heizeinrichtung (10, 10a) aktiviert.

Im Fall F7 liegt die detektierte Temperatur über dem Komfortbereich und die detektierte relative Feuchtigkeit unterhalb des Komfortbereichs. Hierzu wird zur Abkühlung lediglich die Belüftungseinrichtung (11, 11a) aktiviert.

Im Fall F8 liegt die detektierte Temperatur in dem Komfortbereich und die detektierte relative Feuchtigkeit unterhalb des Komfortbereichs. Es werden weder die Heizeinrichtung (10, 10a) noch die Belüftungseinrichtung (11, 11a) aktiviert.

Schließlich liegen im Fall 9 die detektierte relative Feuchtigkeit und die detektierte Temperatur unterhalb des Komfortbereichs. Es wird somit lediglich die Heizeinrichtung (10, 10a) aktiviert.

Ferner kann vorzugsweise eine Vorkonditionierung des Sitzelements (2, 2a, 2b) vor der Sitzbelegung stattfinden (Figur 10). Bei einem vorgebbaren Starterereignis regelt, beziehungsweise steuert die Regel-/Steuereinrichtung (9) die Komfortparameter des Sitzelements (2, 2a, 2b) in dem Komfortbereich. Ein solches Starterereignis kann beispielsweise das Entsperren des Fahrzeugs sein, eine Annäherung des Nutzers an das Fahrzeug, welches durch einen entsprechenden Sensor detektiert wird. Alternativ kann auch eine Einstellung der Komfortparameter in einen Komfortbereich in einer vorgegebenen Zeit erfolgen. Beispielsweise kann die Heizeinrichtung (10, 10a) nach dem Starterereignis aktiviert werden, um die Oberfläche (4, 4a) in den Komfortbereich zu bringen.

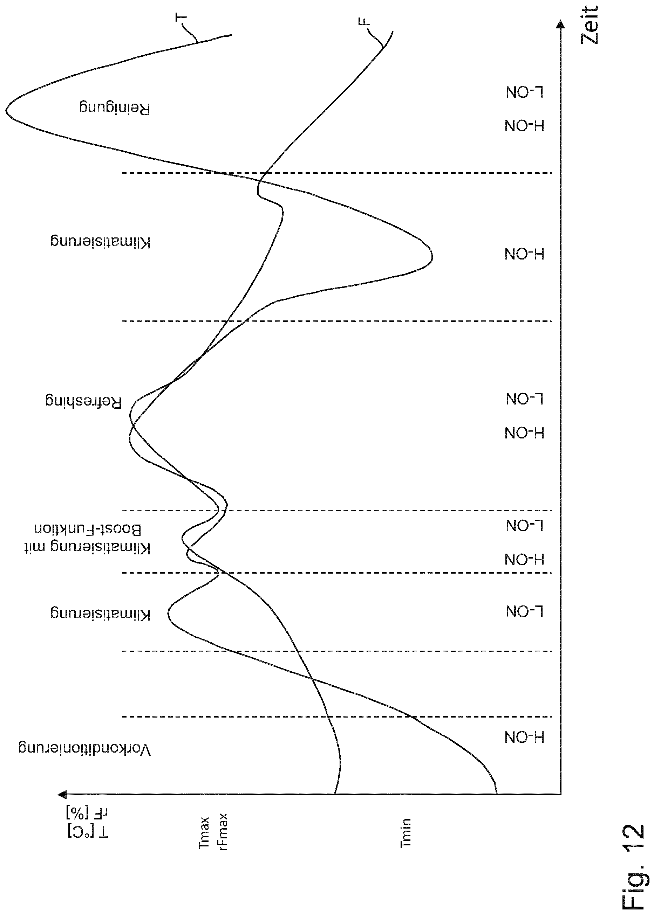

In Figur 12 ist dargestellt, wie sich in den unterschiedlichen Modi die Komfortparameter entsprechend durch das Aktivieren der Heizeinrichtung (10, 10a), beziehungsweise der Belüftungseinrichtung (11, 11a) ändern.

Um die Boost-Funktion besser nutzen zu können, kann es von Vorteil sein, wenn zwei oder mehrere Heizeinrichtungen (10, 10a) in dem Sitzelement (2, 2a, 2b) vorgesehen sind.

Die Modi "Refreshing", Vorkonditionieren und Desinfizieren können auch bei Sitzelementen durchgeführt werden, welche lediglich eine Heizeinrichtung aufweisen.

Ähnlich kann eine Vorkonditionierung auch bei einem Sitzelement (2, 2a, 2b) vorgenommen werden, welche lediglich eine Belüftungseinrichtung (11, 11a, 11b) aufweisen. So kann beispielsweise im Sommer das Sitzelement entsprechend gekühlt werden.

**Bezugszeichenliste**

| 1 | Sitz |
|---|---|
| 2, 2a, 2b | Sitzelement |
| 3, 3a | erste Schicht |
| 4, 4a | Oberfläche |
| 6 | Sensoreinrichtung |
| 7, 7a | Temperatursensor |
| 8, 8a | Feuchtigkeitssensor |
| 9 | Regel-/Steuereinrichtung |
| 9a | Speichereinrichtung |
| 10, 10a | Heizeinrichtung |
| 11, 11a | Belüftungseinrichtung |
| 12, 12a | Abstandsgewirke |
| 13,13a | Formelement |
| 14, 14a | Lüfterbereich |
| 15, 15a | Lüftungskanäle |
| 16 | unteres Sitzteil |
| 17 | Rückenlehnenelement |
| 18, 18a | umrandender Bereich |
| 19 | Sitzbelegungssensor |
| 20 | Verriegelungseinrichtung |

Z   Höhenrichtung des unteres Sitzteils
Z'   Höhenrichtung des Rückenlehnenelements
X   Längsrichtung des unteres Sitzteils
X'   Höhenrichtung des Rückenlehnenelement
Y   Breitenrichtung des unteres Sitzteils
Y'   Breitenrichtung Rückenlehnenelement
h   Höhe der ersten Schicht

**Patentansprüche**

1.  Fahrzeugsitz (1) umfassend zumindest ein Sitzelement (2, 2a, 2b), in welchem zumindest eine Heizeinrichtung (10, 10a) und zumindest eine Belüftungseinrichtung (11, 11a) vorgesehen ist, wobei der Fahrzeugsitz (1) eine Sensoreinrichtung (6) umfasst, welche zumindest einen Temperatursensor (7, 7a) und zumindest einen Feuchtigkeitssensor (8, 8a) aufweist, wobei eine Regel-/Steuereinrichtung (9) anhand der Daten der Sensoreinrichtung (6) bezüglich der Komfortparameter Temperatur und/oder Feuchtigkeitsgehalt die Heizeinrichtung (10, 10a) und/oder die Belüftungseinrichtung (11, 11a) steuert oder regelt, wodurch das Sitzklima aktiv steuerbar oder regelbar ist, **dadurch gekennzeichnet, dass** das zumindest eine Sitzelement (2, 2a, 2b) eine erste Schicht (3, 3a) umfasst, welche aus einem Material besteht, das Feuchtigkeit aufnehmen und durchleiten kann, wobei das Material der ersten Schicht (3, 3a) porös und/oder offenporig ausgebildet ist, wobei das zumindest eine Sitzelement (2, 2a, 2b) durch zumindest eine Heizeinrichtung (10, 10a) aufheizbar ist, wodurch eine in der ersten Schicht (3, 3a) aufgenommene Feuchtigkeit zumindest in einen gasförmigen Zustand übergeht und sich die in dem Sitzelement (2, 2a, 2b) befindliche Luft erwärmt, wobei der durch das Aufheizen erzeugte Dampf mittels der Belüftungseinrichtung aus dem Sitzelement (2, 2a, 2b) beförderbar ist, wobei der Dampf mittels der Belüftungseinrichtung (11, 11a) aus dem Sitzelement (2, 2a, 2b) gesogen wird.

2.  Fahrzeugsitz (1) nach Anspruch 1,
    **dadurch gekennzeichnet, dass**
    die Regel-/Steuereinrichtung (9) eine Speichereinrichtung (9a) aufweist, in welcher Sollwerte oder Sollbereiche für die Komfortparameter oder Sollwerte oder Sollbereiche für Komfortparameterkombinationen abgelegt sind, wobei die Regel-/Steuereinrichtung (9) die durch die Sensoreinrichtung (6) bereitgestellten Daten mit den entsprechenden Sollwerten oder Sollbereichen vergleicht.

3.  Fahrzeugsitz (1) nach Anspruch 1 oder 2,
    **dadurch gekennzeichnet, dass**
    der Fahrzeugsitz (1) zwei Sitzelementen (2, 2a, 2b) umfasst, wobei ein Sitzelement (2a) ein unteres Sitzteil (16) ist und das weitere Sitzelement (2, 2b) ein Rückenlehnenelement (17) ist, wobei die Sensoreinrichtung (6) zumindest einen Temperatursensor (7) und zumindest einen Feuchtigkeitssensor (8) für das untere Sitzteil (16) und zumindest einen Temperatursensor (7a) und zumindest einen Feuchtigkeitssensor (8a) für das Rückenlehnenelement (17) umfasst.

4.  Fahrzeugsitz nach einem der vorhergehenden Ansprüche,
    **dadurch gekennzeichnet, dass**
    bei Sitzbelegung eine Oberfläche (4, 4a) der ersten Schicht (3, 3a) bei Sitzbelegung in direktem Kontakt mit dem Nutzer oder in unmittelbarer Nähe zu dem Nutzer ist.

5.  Fahrzeugsitz nach einem der vorhergehenden Ansprüche,
    **dadurch gekennzeichnet, dass**
    der zumindest eine Temperatursensor (7, 7a) und/oder der zumindest eine Feuchtigkeitssensor (8, 8a) in dem zumindest einem Sitzelement (2, 2a, 2b) derart angeordnet sind, dass die Komfortparameter Temperatur und/oder Feuchtigkeit an oder nahe der Oberfläche (4, 4a) des zumindest einem Sitzelements (2, 2a, 2b) erfassbar sind, wobei der zumindest eine Temperatursensor (7, 7a) und/oder der zumindest eine Feuchtigkeitssensor (8, 8a) in und/oder über und/oder unter der ersten Schicht (3, 3a) angeordnet ist, wobei die Regel-/ Steuereinrichtung (9) die von der Sensoreinrichtung (6) übertragenen Daten in Abhängigkeit zum Abstand des jeweiligen Sensors (7, 7a, 8, 8a) zu der mit dem Nutzer in Kontakt stehenden Oberfläche (4, 4a) des zumindest einen Sitzelements (2, 2a, 2b) ausgewertet werden.

6.  Fahrzeugsitz nach einem der Ansprüche 4 bis 5,
    **dadurch gekennzeichnet, dass**
    in Höhenrichtung (Z, Z') des zumindest einen Sitzelements (2, 2a, 2b) unter der ersten Schicht (3, 3a) ein Abstandsgewirke (12, 12a) angeordnet ist, wobei zwischen der ersten Schicht (3, 3a) und dem Abstandsgewirke (12, 12a) eine Heizeinrichtung (10, 10a) angeordnet ist.

7.  Fahrzeugsitz nach Anspruch 6,
    **dadurch gekennzeichnet, dass**
    in Höhenrichtung (Z, Z') des zumindest einen Sitzelements (2, 2a, 2b) unter dem Abstandsgewirke (12, 12a) zumindest abschnittsweise ein Formelement (13, 13a) angeordnet ist, welches einen Lüfterbereich (14, 14a) aufweist, in welchem eine Vielzahl an Lüftungskanälen (15, 15a) vorgesehen ist, wobei die Lüftungskanäle (15, 15a) mit der zumindest einen Belüftungseinrichtung (11, 11a) verbunden sind, wobei das Formelement (13, 13a) einen den Lüfterbereich (14, 14a) umrandenden Bereich (18, 18a) aufweist.

8. Fahrzeugsitz (1) nach einem der vorhergehenden Ansprüche,
   **dadurch gekennzeichnet, dass**
   durch die Aggregatszustandsveränderung sich, aufgrund der aufzubringenden Verdampfungswärme, eine Kühlung der Oberfläche des Sitzelements (2, 2a, 2b) ergibt, wobei durch die erhöhte Temperatur des Dampfes ein Differenzdruck bezüglich des umliegenden Luftvolumens vorherrscht, welcher das Entfernen des Dampfes begünstigt.

9. Fahrzeugsitz nach einem der vorhergehenden Ansprüche,
   **dadurch gekennzeichnet, dass**
   die Sensoreinrichtung (6) einen Sitzbelegungssensor (19) umfasst, welcher die Sitzbelegung detektiert.

10. Fahrzeugsitz nach einem der vorhergehenden Ansprüche,
    **dadurch gekennzeichnet, dass**
    die Regelung des Sitzklimas durch abwechselndes Heizen und Belüften der ersten Schicht (3, 3a) erfolgt, wobei die zumindest eine Heizeinrichtung (10, 10a) und/oder die zumindest eine Belüftungseinrichtung (11, 11a) in vorgegebenen Intervallen ein-, beziehungsweise ausschaltbar sind, wobei die zumindest eine Heizeinrichtung (10, 10a) derart in Intervallen schaltbar ist, dass an der Oberfläche (4, 4a) des Sitzelements (2, 2a, 2b) im Wesentlichen keine Temperaturänderung erfolgt.

11. Verfahren zum Betrieb eines Fahrzeugsitzes (1), insbesondere einem Fahrzeugsitz nach einem der vorhergehenden Ansprüche 1 bis 9, wobei der Fahrzeugsitz (1) zumindest ein Sitzelement (2), in welchem zumindest eine Heizeinrichtung (10) und zumindest eine Belüftungseinrichtung (11) vorgesehen ist umfasst, wobei das Verfahren folgende Verfahrensschritte umfasst:

    a) Erfassen von Sensordaten durch die Sensoreinrichtung (6), welche zumindest einen Temperatursensor (7) und zumindest einen Feuchtigkeitssensor (8) aufweist;
    b) Optional vergleichen der erfassten Sensordaten mit vorgegebenen Sollwerten durch die Regel-/Steuereinrichtung (9);
    c) Aktivieren zumindest einer Heizeinrichtung (10, 10a) und/oder zumindest einer Belüftungseinrichtung (11, 11a) durch die Regel-/Steuereinrichtung (9), wobei das zumindest eine Sitzelement (2, 2a, 2b) eine erste Schicht (3, 3a) umfasst, welche aus einem Material besteht, das Feuchtigkeit aufnehmen und durchleiten kann sowie das Material der ersten Schicht (3, 3a) porös und/oder offenporig ausgebildet ist, wobei das zumindest eine Sitzelement (2, 2a, 2b) durch zumindest eine Heizeinrichtung (10, 10a) aufheizbar ist, wodurch eine in der ersten Schicht (3, 3a) aufgenommene Feuchtigkeit zumindest in einen gasförmigen Zustand übergeht und sich die in dem Sitzelement (2, 2a, 2b) befindliche Luft erwärmt, wobei der durch das Aufheizen erzeugte Dampf mittels der Belüftungseinrichtung aus dem Sitzelement (2, 2a, 2b) beförderbar ist, wobei der Dampf mittels der Belüftungseinrichtung (11, 11a) aus dem Sitzelement (2, 2a, 2b) gesogen wird.

12. Verfahren nach Anspruch 11,
    **dadurch gekennzeichnet, dass**
    bei einer nicht vorhandenen Sitzbelegung durch die Regel-/Steuereinrichtung (9) eine antibakterielle Reinigung des Sitzelements ausgeführt wird, in dem die zumindest eine Heizeinrichtung (10) angesteuert wird, so dass das zumindest eine Sitzelement auf eine Temperatur gebracht wird, bei welcher Bakterien und Keime abgetötet werden, wobei diese Temperatur über 80°C beträgt.

13. Verfahren nach einem der Ansprüche 11 bis 12
    **dadurch gekennzeichnet, dass**
    bei einer vorhandenen Sitzbelegung durch die Regel-/Steuereinrichtung (9) die zumindest eine Heizeinrichtung (10, 10a) und die zumindest eine Belüftungseinrichtung (11, 11a) in abwechselnden Intervallen aktiviert werden, wobei die Intervalllänge für eine Aktivierung der zumindest einen Heizeinrichtung (10, 10a) derart ausgelegt ist, dass an der Oberfläche (4, 4a) des Sitzelements (2, 2a, 2b) im Wesentlichen keine Temperaturänderung erfolgt.

14. Verfahren nach einem der Ansprüche 11 bis 13,
    **dadurch gekennzeichnet, dass**
    bei einer nicht vorhandenen Sitzbelegung durch die Regel-/Steuereinrichtung (9) die zumindest eine Heizeinrichtung (10, 10a) und die zumindest eine Belüftungseinrichtung (11, 11a) in abwechselnden Intervallen aktiviert werden, wobei die Intervalllänge für eine Aktivierung der zumindest einen Heizeinrichtung (10, 10a) derart ausgelegt ist, dass das zumindest eine Sitzelement auf eine Temperatur erwärmt wird, welche über einer vorgegebenen Komfortbereich liegt, wobei der Komfortbereich der Temperatur zwischen 28°C und 38°C liegt.

15. Verfahren nach einem der Ansprüche 11 bis 14,
    **dadurch gekennzeichnet, dass**
    bei Eintritt eines vorgegebenen Startereignis eine Vorkonditionierung des zumindest einen Sitzelements (2, 2a, 2b) erfolgt, in welcher durch Aktivierung der zumindest einen Heizeinrichtung (10, 10a) und/oder der zumindest einen Belüftungseinrichtung (11, 11a) durch die Regel-/Steuereinrichtung (9) die Komfortparameter in den vorgegebenen Komfortbereich gebracht werden.

**Claims**

1. Vehicle seat (1) comprising at least one seat element (2, 2a, 2b) in which at least one heating device (10, 10a) and at least one ventilation device (11, 11a) is provided, wherein the vehicle seat (1) comprises a sensor device (6) which has at least one temperature sensor (7, 7a) and at least one humidity sensor (8, 8a), wherein an open-loop/closed-loop control device (9) controls the heating device (10, 10a) and/or the ventilation device (11, 11a) on the basis of the data of the sensor device (6) with respect to the comfort parameters temperature and/or humidity content, whereby the seat climate can be actively controlled,
**characterised in that**
the at least one seat element (2, 2a, 2b) comprises a first layer (3, 3a) which consists of a material which can absorb and transmit moisture, wherein the material of the first layer (3, 3a) is porous and/or open-pored, wherein the at least one seat element (2, 2a, 2b) can be heated by at least one heating device (10, 10a), whereby moisture absorbed in the first layer (3, 3a) changes at least into a gaseous state and the air located in the seat element (2, 2a, 2b) is heated, wherein the vapour generated by the heating can be conveyed out of the seat element (2, 2a, 2b) by means of the ventilation device, wherein the vapour is drawn out of the seat element (2, 2a, 2b) by means of the ventilation device (11, 11a).

2. Vehicle seat (1) according to claim 1,
**characterised in that**
the open-loop/closed-loop control device (9) has a memory device (9a), in which setpoint values or setpoint range e for the comfort parameters or setpoint values or setpoint ranges for comfort parameter combinations are stored , wherein the open-loop/closed-loop control device (9) compares the data provided by the sensor device (6) with the corresponding setpoint values or setpoint ranges.

3. Vehicle seat (1) according to claim 1 or 2,
**characterised in that**
the vehicle seat (1) comprises two seat elements (2, 2a, 2b), wherein one seat element (2a) is a lower seat piece (16) and the other seat element (2, 2b) is a backrest element (17), wherein the sensor device (6) comprises at least one temperature sensor (7) and at least one humidity sensor (8) for the lower seat piece (16) and at least one temperature sensor (7a) and at least one humidity sensor (8a) for the backrest element (17).

4. Vehicle seat according to one of the preceding claims,
**characterised in that**
a surface (4, 4a) of the first layer (3, 3a) is in direct contact with the user or in close proximity to the user when the seat is occupied.

5. Vehicle seat according to one of the preceding claims,
**characterised in that**
the at least one temperature sensor (7, 7a) and/or the at least one humidity sensor (8, 8a) are arranged in the at least one seat element (2, 2a, 2b) in such a way that the comfort parameters temperature and/or humidity can be detected at or near the surface (4, 4a) of the at least one seat element (2, 2a, 2b), wherein the at least one temperature sensor (7, 7a) and/or the at least one humidity sensor (8, 8a) is arranged in and/or above and/or below the first layer (3, 3a), wherein the open-loop/closed-loop control device (9) analyses the data transmitted by the sensor device (6) as a function of the distance of the respective sensor (7, 7a, 8, 8a) to the surface (4, 4a) of the at least one seat element (2, 2a, 2b) that is in contact with the user.

6. Vehicle seat according to one of claims 4 to 5,
**characterised in that**
a knitted spacer fabric (12, 12a) is arranged under the first layer (3, 3a) in the height direction (Z, Z') of the at least one seat element (2, 2a, 2b) , wherein a heating device (10, 10a) is arranged between the first layer (3, 3a) and the knitted spacer fabric (12, 12a).

7. Vehicle seat according to claim 6,
**characterised in that**
in the height direction (Z, Z') of the at least one seat element (2, 2a, 2b) a shaped piece (13, 13a) is arranged at least in sections under the knitted spacer fabric (12, 12a), which shaped piece has a ventilator region (14, 14a), in which a plurality of ventilation ducts (15, 15a) is provided, wherein the ventilation ducts (15, 15a) are connected to the at least one ventilation device (11, 11a), wherein the shaped piece (13, 13a) has a region (18, 18a) surrounding the fan region (14, 14a).

8. Vehicle seat (1) according to one of the preceding claims,
**characterised in that**
the change in aggregate state results in a cooling of the surface of the seat element (2, 2a, 2b) due to the heat of vaporisation to be applied, wherein a differential pressure prevails with respect to the surrounding air volume due to the increased temperature of the vapour, which favours the removal of the vapour.

9. Vehicle seat according to one of the preceding claims,
**characterised in that**
the sensor device (6) comprises a seat occupancy sensor (19), which detects the seat occupancy.

10. Vehicle seat according to one of the preceding claims,
**characterised in that**
the seat climate is controlled by alternately heating and ventilating the first layer (3, 3a), wherein the at least one heating device (10, 10a) and/or the at least one ventilating device (11, 11a) can be switched on or off at predetermined intervals, wherein the at least one heating device (10, 10a) can be switched at intervals in such a way that essentially no temperature change occurs on the surface (4, 4a) of the seat element (2, 2a, 2b) .

11. Method for operating a vehicle seat (1), in particular a vehicle seat according to one of the preceding claims 1 to 9, wherein the vehicle seat (1) comprises at least one seat element (2), in which at least one heating device (10) and at least one ventilation device (11) is provided, wherein the method comprises the following method steps:

a) Acquisition of sensor data by the sensor device (6), which has at least one temperature sensor (7) and at least one humidity sensor (8);
b) Optional comparison of the recorded sensor data with predetermined target values by the open-loop/closed-loop control device (9);
c) Activation of at least one heating device (10, 10a) and/or at least one ventilation device (11, 11a) by the open-loop/closed-loop control device (9), wherein the at least one seat element (2, 2a, 2b) comprises a first layer (3, 3a) which consists of a material which can absorb and transmit moisture and the material of the first layer (3, 3a) is porous and/or open-pored, wherein the at least one seat element (2, 2a, 2b) can be heated by at least one heating device (10, 10a), whereby moisture absorbed in the first layer (3, 3a) changes at least into a gaseous state and the air located in the seat element (2, 2a, 2b) is heated, wherein the vapour generated by the heating can be conveyed out of the seat element (2, 2a, 2b) by means of the ventilation device, wherein the vapour is drawn out of the seat element (2, 2a, 2b) by means of the ventilation device (11, 11a).

12. Method according to claim 11, **characterised in that**
when the seat is not occupied, an antibacterial cleaning of the seat element is carried out by the open-loop/closed-loop control device (9), in which the at least one heating device (10) is activated so that the at least one seat element is brought to a temperature at which bacteria and germs are killed, this temperature being above 80°C .

13. Method according to one of claims 11 to 12
**characterised in that**
when the seat is occupied, the at least one heating device (10, 10a) and the at least one ventilation device (11, 11a) are activated by the open-loop/-closed-loop control device (9) at alternating intervals, the interval length for activation of the at least one heating device (10, 10a) being designed such that essentially no temperature change takes place on the surface (4, 4a) of the seat element (2, 2a, 2b).

14. Method according to one of claims 11 to 13,
**characterised in that**
when the seat is not occupied, the at least one heating device (10, 10a) and the at least one ventilation device (11, 11a) are activated by the open-loop/-closed-loop control device (9) at alternating intervals, wherein the interval length for activation of the at least one heating device (10, 10a) is designed such that the at least one seat element is heated to a temperature which is above a predetermined comfort range, wherein the comfort range of the temperature is between 28°C and 38°C.

15. Method according to one of claims 11 to 14, **characterised in that**
a preconditioning of the at least one seat element (2, 2a, 2b) takes place when a predetermined start event occurs, in which preconditioning the comfort parameters are brought into the predetermined comfort range by activating the at least one heating device (10, 10a) and/or the at least one ventilation device (11, 11a) by the open-loop/closed-loop control device (9).

**Revendications**

1. Siège de véhicule (1) comportant au moins un élément de siège (2, 2a, 2b), dans lequel sont disposés au moins un dispositif de chauffage (10, 10a) et au moins un dispositif de ventilation (11, 11a), dans lequel le siège de véhicule (1) comporte un dispositif de détection (6), lequel présente au moins un capteur de température (7, 7a) et au moins un capteur d'humidité (8, 8a), dans lequel un dispositif de régulation/de commande (9) commande ou régule le dispositif de chauffage (10, 10a) et/ou le dispositif de ventilation (11, 11a) à l'aide des données provenant du dispositif de détection (6) en ce qui concerne les paramètres de confort que sont la température et/ou la teneur en humidité, ce par quoi la climatisation du siège est commandable ou régulable activement,

**caractérisé par le fait que** ledit au moins un élément de siège (2, 2a, 2b) comporte une première couche (3, 3a), laquelle est constituée d'un matériau qui peut absorber et évacuer l'humidité, le matériau de la première couche (3, 3a) étant poreux et/ou à pores ouverts, ledit au moins un élément de siège (2, 2a, 2b) étant apte à être chauffé par au moins un dispositif de chauffage (10, 10a), ce par quoi une humidité absorbée dans la première couche (3, 3a) passe au moins dans un état gazeux et l'air se trouvant dans l'élément de siège (2, 2a, 2b) se réchauffe, la vapeur produite par le chauffage étant apte à être transportée hors de l'élément de siège (2, 2a, 2b) au moyen du dispositif de ventilation, la vapeur étant aspirée hors de l'élément de siège (2, 2a, 2b) au moyen du dispositif de ventilation (11, 11a).

2. Siège de véhicule (1) selon la revendication 1, **caractérisé par le fait que** le dispositif de régulation/de commande (9) présente un dispositif de stockage (9a) dans lequel sont stockées des valeurs cibles ou des plages cibles pour les paramètres de confort ou des valeurs cibles ou des plages cibles pour des combinaisons de paramètres de confort, le dispositif de régulation/de commande (9) comparant les données fournies par le dispositif de détection (6) avec les valeurs cibles ou plages cibles correspondantes.

3. Siège de véhicule (1) selon l'une des revendications 1 ou 2, **caractérisé par le fait que** le siège de véhicule (1) comporte deux éléments de siège (2, 2a, 2b), un élément de siège (2a) étant une partie siège inférieure (16) et l'autre élément de siège (2, 2b) étant un élément dossier (17), le dispositif de détection (6) comportant au moins un capteur de température (7) et au moins un capteur d'humidité (8) pour la partie siège inférieure (16) et au moins un capteur de température (7a) et au moins un capteur d'humidité (8a) pour l'élément dossier (17).

4. Siège de véhicule selon l'une des revendications précédentes, **caractérisé par le fait que** lorsque le siège est occupé, une surface (4, 4a) de la première couche (3, 3a) est en contact direct avec l'utilisateur ou à proximité immédiate de l'utilisateur.

5. Siège de véhicule selon l'une des revendications précédentes, **caractérisé par le fait que** ledit au moins un capteur de température (7, 7a) et/ou ledit au moins un capteur d'humidité (8, 8a) sont disposés dans ledit au moins un élément de siège (2, 2a, 2b) de telle sorte que les paramètres de confort que sont la température et/ou l'humidité sont détectables sur ou à proximité de la surface (4, 4a) dudit au moins un élément de siège (2, 2a, 2b), ledit au moins un capteur de température (7, 7a) et/ou ledit au moins un capteur d'humidité (8, 8a) étant disposé(s) dans et/ou sur et/ou sous la première couche (3, 3a), le dispositif de régulation/de commande (9) évaluant les données transmises par le dispositif de détection (6) en fonction de la distance du capteur respectif (7, 7a, 8, 8a) à la surface (4, 4a), en contact avec l'utilisateur, dudit au moins un élément de siège (2, 2a, 2b).

6. Siège de véhicule selon l'une des revendications 4 et 5, **caractérisé par le fait que** dans la direction de la hauteur (Z, Z') dudit au moins un élément de siège (2, 2a, 2b), un tissu d'espacement (12, 12a) est disposé sous la première couche (3, 3a), un dispositif de chauffage (10, 10a) étant disposé entre la première couche ( 3, 3a) et le tissu d'espacement (12, 12a).

7. Siège de véhicule selon la revendication 6, **caractérisé par le fait que** dans la direction de la hauteur (Z, Z') dudit au moins un élément de siège (2, 2a, 2b), un élément façonné (13, 13a) est disposé au moins par sections sous le tissu d'espacement (12, 12a), lequel élément façonné présente une zone de ventilation (14, 14a) dans laquelle une pluralité de canaux de ventilation (15, 15a) est prévue, les canaux de ventilation (15, 15a) étant reliés audit au moins un dispositif de ventilation (11, 11a), l'élément façonné (13, 13a) présentant une zone (18, 18a) bordant la zone de ventilation (14, 14a).

8. Siège de véhicule (1) selon l'une des revendications précédentes, **caractérisé par le fait que** le changement d'état physique conduit, en raison de la chaleur d'évaporation à appliquer, à un refroidissement de la surface de l'élément de siège (2, 2a, 2b), une pression différentielle prédominant en ce qui concerne le volume d'air environnant en raison de la température augmentée de la vapeur, laquelle pression facilite le retrait de la vapeur.

9. Siège de véhicule selon l'une des revendications précédentes, **caractérisé par le fait que** le dispositif de détection (6) comporte un capteur d'occupation de siège (19), lequel détecte l'occupation du siège.

10. Siège de véhicule selon l'une des revendications précédentes, **caractérisé par le fait que**

la régulation de la climatisation du siège s'effectue par chauffage et ventilation en alternancs de la première couche (3, 3a), ledit au moins un dispositif de chauffage (10, 10a) et/ou ledit au moins un dispositif de ventilation (11, 11a) étant activables ou désactivables à intervalles prédéfinis, ledit au moins un dispositif de chauffage (10, 10a) étant commutable à intervalles de telle sorte qu'il n'y a sensiblement pas de changement de température à la surface (4, 4a) de l'élément de siège (2, 2a, 2b).

11. Procédé de fonctionnement d'un siège de véhicule (1), en particulier d'un siège de véhicule selon l'une des revendications précédentes 1 à 9, dans lequel le siège de véhicule (1) comporte au moins un élément de siège (2), dans lequel sont disposés au moins un dispositif de chauffage (10) et au moins un dispositif de ventilation (11), le procédé comportant les étapes suivantes :

a) Saisie de données de détection par le dispositif de détection (6), lequel présente au moins un capteur de température (7) et au moins un capteur d'humidité (8) ;
b) Comparaison facultative par le dispositif de régulation/de commande (9) des données de détection saisies avec des valeurs cibles prédéfinies ;
c) Activation d'au moins un dispositif de chauffage (10, 10a) et/ou d'au moins un dispositif de ventilation (11, 11a) par le dispositif de régulation/de commande (9), ledit au moins un élément de siège (2, 2a, 2b) comportant une première couche (3, 3a), laquelle est constituée d'un matériau qui peut absorber et évacuer l'humidité et le matériau de la première couche (3, 3a) étant réalisé poreux et/ou à pores ouverts, ledit au moins un élément de siège (2, 2a, 2b) étant apte à être chauffé par au moins un dispositif de chauffage (10, 10a), ce par quoi une humidité absorbée dans la première couche (3, 3a) passe au moins dans un état gazeux et l'air se trouvant dans l'élément de siège (2, 2a, 2b) se réchauffe, la vapeur produite par le chauffage étant apte à être transportée hors de l'élément de siège (2, 2a, 2b) au moyen du dispositif de ventilation, la vapeur étant aspirée hors de l'élément de siège (2, 2a, 2b) au moyen du dispositif de ventilation (11, 11a).

12. Procédé selon la revendication 11, **caractérisé par le fait que**
lorsque le siège n'est pas occupé, le dispositif de régulation/de commande (9) effectue un nettoyage antibactérien de l'élément de siège, dans lequel ledit au moins un dispositif de chauffage (10) est commandé, de telle sorte que ledit au moins un élément de siège est amené à une température à laquelle les bactéries et les germes sont tués, cette température étant supérieure à 80°C.

13. Procédé selon l'une des revendications 11 et 12, **caractérisé par le fait que**
lorsque le siège est occupé, ledit au moins un dispositif de chauffage (10, 10a) et ledit au moins un dispositif de ventilation (11, 11a) sont activés par le dispositif de régulation/de commande (9) à intervalles alternés, la longueur d'intervalle pour une activation dudit au moins un dispositif de chauffage (10, 10a) étant conçue de telle sorte qu'il n'y a sensiblement pas de changement de température sur la surface (4, 4a) de l'élément de siège (2, 2a, 2b).

14. Procédé selon l'une des revendications 11 à 13, **caractérisé par le fait que**
lorsque le siège n'est pas occupé, ledit au moins un dispositif de chauffage (10, 10a) et ledit au moins un dispositif de ventilation (11, 11a) sont activés par le dispositif de régulation/de commande (9) à intervalles alternés, la longueur d'intervalle pour une activation dudit au moins un dispositif de chauffage (10, 10a) étant conçue de telle sorte que ledit au moins un élément de siège est chauffé à une température qui est supérieure à une plage de confort prédéterminée, la plage de confort de la température étant entre 28°C et 38°C.

15. Procédé selon l'une des revendications 11 à 14, **caractérisé par le fait que**
lors de l'apparition d'un événement de démarrage prédéfini, a lieu un préconditionnement dudit au moins un élément de siège (2, 2a, 2b), dans lequel les paramètres de confort sont amenés dans la plage de confort prédéfinie par l'activation dudit au moins un dispositif de chauffage (10, 10a) et/ou dudit au moins un dispositif de ventilation (11, 11a) par le dispositif de régulation/de commande (9).

Fig. 1a

Fig. 1b

Fig. 1c

Fig. 2

EP 3 844 021 B1

| Feuchtigkeit | | Temperatur |
|---|---|---|

① Zustände

80% ———————————————————————— 38°

② **Komfortbereich des Fahrers** 65% ———————————————————————— 35,5°

50% ———————————————————————— 28°

③

## Fig. 3

Sitz ist überhitzt

Fahrer setzt sich auf den Sitz

Fahrer überhitzt durch den Körperkontakt zum Sitz

Fahrer fängt zum schwitzen an, Schweißabgabe an den Sitz

Sitz wird Feucht / Nass

Sitz wird belüftet, abwechselnd, geheizt

Trocknungsprogramm /Boost Funktion

Ziel Temperatur/T.

T= 35,5 Grad

Relative Feuchtigkeit / r.F.

Ziel: 65% r.F.

Fig. 4

```
┌─────────────────────────┐
│      Sitz ist kalt      │
└─────────────────────────┘
              │
              ▼
┌─────────────────────────────┐
│ Fahrer setzt sich auf den Sitz │
└─────────────────────────────┘
              │
              ▼
```

| Fahrer unterkühlt durch den Körperkontakt zum Sitz | → | Fahrer fängt zum Frieren an | → | Sitz wird langsam warm durch den Fahrer |

```
                                                                        │
                                                                        ▼
                                                          ┌─────────────────────┐
                                                          │   Sitz wird geheizt  │
                                                          └─────────────────────┘
```

Fig. 5

Sitzbelegung

Desinfektion

Vorkonditionierung

nein — nein — ja

**Modus: Reinigung**

| Heizung ON | Lüfter ON |

**Modus: Refreshing**

rF [%]

> rFmax

ja

| Heizung ON | Lüfter ON |

**Modus: Klimatisierung**

T [°C]

< Tmin — nein — > Tmax

ja — ja

boost-Funktion

> rFmax

ja

| Heizung ON | Lüfter ON |

Fig. 6

Fig. 7

Fig. 8

Fig. 9

Fig. 10

**Mögliche Fälle:**

**Aufteilung der Funktionsfälle:**

| Temperatur | | Feuchtigkeit | |
|---|---|---|---|
| 1 | Hoch | 1 | Hoch |
| 2 | Komfort | 2 | Komfort |
| 3 | Nieder | 3 | Nieder |

| | | | |
|---|---|---|---|
| **F1** | T1/RF1 | L-ON | **H-OFF** |
| **F2** | T2/RF1 | L-ON | **L-ON** |
| **F3** | T3/RF1 | L-ON | **H-ON** |
| **F4** | T1/RF2 | L-ON | **H-OFF** |
| **F5** | T2/RF2 | L-OFF | **H-OFF** |
| **F6** | T3/RF2 | L-OFF | **H-ON** |
| **F7** | T1/RF3 | L-ON | **H-OFF** |
| **F8** | T2/RF3 | L-OFF | **H-OFF** |
| **F9** | T3/RF3 | L-OFF | **H-ON** |

**Boost Funktion**

H-ON nur so lange das die eingeleitete Wärme vom Fahrer nicht wahrgenommen wird, zu vor H-OFF und L-On Im Wechsel oder Gleichzeitig an.

Legende: F1-F9 sind die Fälle, T1-T3 sind die Zustände siehe Diagramm,L-ON / L-OFF an-aus, H-ON / H-OFF ist Heizung an-aus

Fig. 11

EP 3 844 021 B1

Fig. 12